(19) Europäisches Patentamt
European Patent Office
Office européen des brevets

(11) Publication number: **0 668 081 A2**

(12) **EUROPEAN PATENT APPLICATION**

(21) Application number: **95101927.2**

(22) Date of filing: **13.02.95**

(51) Int. Cl.[6]: **A61L 27/00**, C08L 89/06, C08G 81/00, C08H 1/06

(30) Priority: **17.02.94 US 201860**

(43) Date of publication of application: **23.08.95 Bulletin 95/34**

(84) Designated Contracting States: **AT CH DE ES FR GB IT LI SE**

(71) Applicant: **COLLAGEN CORPORATION**
**2500 Faber Place**
**Palo Alto, California 94303-3334 (US)**

(72) Inventor: **Rhee, Woonza M.**
**3845 LaDonna Avenue**
**Palo Alto, Ca 94306 (US)**

(74) Representative: **Schwan, Gerhard, Dipl.-Ing.**
**Elfenstrasse 32**
**D-81739 München (DE)**

(54) **Collagen-synthetic polymer conjugates having controlled fiber size distributions.**

(57) Current commercially available fibrillar collagen compositions tend to have heterogeneous fiber size populations, including very large fibers, which are not conducive to efficient crosslinking using chemical crosslinking agents. The present invention discloses preferred methods for producing collagens having relatively homogeneous, controlled fiber size populations, which are then covalently conjugated to synthetic hydrophilic polymers, such as functionally activated polymeric glycols, to produce collagen-synthetic polymer conjugates having unique physical and chemical characteristics. The resulting conjugates are used to prepare formed implants or injectable formulations for use in a variety of therapeutic applications. Compositions of the conjugates may include additional components, such as pharmaceutically acceptable fluid carriers and/or biologically active molecules such as growth factors or cytokines.

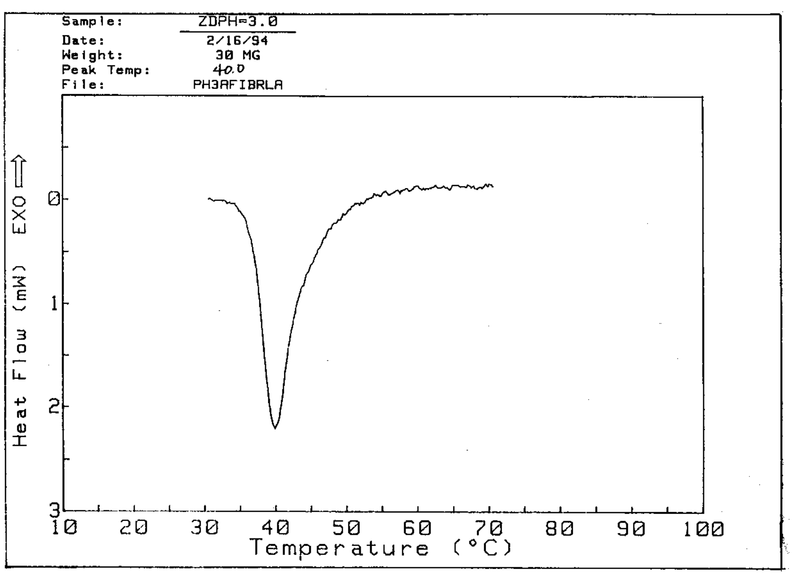


Figure 1.

EP 0 668 081 A2

## Field of the Invention

This invention relates generally to collagens having controlled fiber size distributions which are covalently conjugated to synthetic hydrophilic polymers, such as functionally activated polymeric glycols, to produce crosslinked collagen compositions having unique physical and chemical characteristics. Specifically, the invention relates to improved collagen-synthetic polymer conjugates wherein at least 80% of the collagen fibers have a fiber size of about 10 microns or less. The improved collagen-synthetic polymer conjugates can be used to produce formed implants for use in a variety of therapeutic applications. Compositions of the conjugates may also include other components, such as pharmaceutically acceptable fluid carriers to form injectable formulations, and/or biologically active molecules such as growth factors or cytokines. Methods for preparing the conjugates are also disclosed.

## Background of the Invention

Daniels et al., U.S. Pat. No. 3,949,073, disclosed the preparation of soluble collagen by dissolving tissue in aqueous acid, followed by enzymatic digestion. The resulting atelopeptide collagen is soluble, and substantially less immunogenic than unmodified collagen. It may be injected into suitable locations of a subject with a fibril-formation promoter (described as a polymerization promoter in the patent) to form fibrous collagen implants *in situ*, for augmenting hard or soft tissue. This material is now commercially available from Collagen Corporation (Palo Alto, CA) under the trademark Zyderm® Collagen Implant.

Davis et al., U.S. Pat. 4,179,337, disclosed a physiologically active, water-soluble polypeptide composition comprising a physiologically active polypeptide coupled with a coupling agent to polyethylene glycol or polypropylene glycol.

Chvapil, U.S. Pat. Nos. 4,193,813 and 4,274,410, disclosed a method for making a collagen sponge comprising comminuting and homogenizing native fibrillar collagen in an acid environment, removing noncollagenous material and mixing the residual collagen with water, adjusting the pH of the resulting slurry to 4.5 to 5.0 by adding acetic acid, adding glutaraldehyde, then pouring the slurry into molds and freezing at approximately -10°C for approximately 20 hours. The frozen mass is then thawed, washed, and immersed in a wash of pH 8 - 9 for approximately 2 hours at 20°C. Sufficient reducing agent is added to the wash to create excess reducing equivalents. The sponge is then immersed in a buffer solution of pH 4 - 5 for a time sufficient to equilibrate to uniform pH.

DeVore et al., U.S. Pat. No. 4,242,291, disclosed precipitation of tropocollagen molecules into collagen fibrils and aggregation of the fibrils into collagen gels using conventional methods at less than one gravity, preferably zero gravity, to produce collagen aggregates having better properties than aggregates produced at normal earth gravity.

Cioca et al., U.S. Pat. No. 4,295,894, disclosed a method of forming soluble collagen fibers from animal hides by treating the animal hides with an aqueous solution of an alkali metal hydroxide and a dehydrating agent to remove the hair and fat. The interfibrillar bonds of the collagen in the corium are stabilized with an aqueous solution of an alkali sulfate. The corium is neutralized and dissolved in an aqueous acid solution to yield a collagen solution. The pH of the collagen solution is adjusted to the isoelectric point of the collagen to precipitate the collagen fibers from the solution, which are then dried. The collagen fibers produced may be redissolved and further processed for a variety of uses.

Luck et al., U.S. Pat. No. 4,488,911, disclosed a method for preparing collagen in solution (CIS), wherein native collagen is extracted from animal tissue in dilute aqueous acid, followed by digestion with an enzyme such as pepsin, trypsin, or Pronase® (a trademark of American Hoechst Corporation, Somerville, NJ). The enzymatic digestion removes the telopeptide portions of the collagen molecules, providing "atelopeptide" collagen in solution. The atelopeptide collagen in solution so produced is substantially nonimmunogenic, and is also substantially non-crosslinked due to loss of the primary crosslinking regions. The collagen in solution may then be precipitated by dialysis in a moderate shear environment to produce collagen fibers which resemble native collagen fibers. The precipitated, reconstituted fibers may additionally be crosslinked using a chemical agent (for example, aldehydes such as formaldehyde and glutaraldehyde), heat, or radiation. The resulting products are suitable for use in medical implants due to their biocompatability and reduced immunogenicity.

Chu, U.S. Pat. No. 4,557,764, disclosed a "second nucleation" collagen precipitate which exhibits a desirable malleability and putty-like consistency. Collagen is provided in solution (*e.g.*, at 2 - 4 mg/ml), and a "first nucleation product" is precipitated by rapid titration and centrifugation. The remaining supernatant (containing the bulk of the original collagen) is then decanted and allowed to stand overnight. The precipitated second nucleation product is collected by centrifugation.

Chu, U.S. Pat. Nos. 4,600,533; 4,655,980; 4,689,399; and 4,725,617, disclosed methods for preparing collagen membranes having high tensile strength by compressing and drying collagen gels.

Nguyen et al., U.S. Pat. No. 4,642,117, disclosed an injectable collagen material composed of reconstituted, mechanically sheared atelopeptide collagen fibers, which are prepared by passing reconstituted colalgen fibers repeatedly through a rigid mesh screen, untail a substantial reduction in fiber size and size heterogeneity is achieved. The mechanically sheared fibers may be subsequently crosslinked.

Beutler et al., U.S. Pat. No. 4,789,401, disclosed a non-crosslinked collagen sponge which is prepared from an optically clear collagen solution by precipitation with neutral salts, dissolving and reprecipitation, and then subjecting the collagen to freeze-drying without removal of the precipitation salt.

Ramshaw et al., U.S. Pat. No. 4,980,403, disclosed the precipitation of bovine collagen (types I, II, and III) from aqueous PEG solutions, where there is no binding between collagen and PEG.

Goldstein, U.S. Pat. No. 5,043,426, disclosed a process for manufacturing organized collagen structures, which may be either fibers or membranes, by grinding animal or human tissue in saline solution in order to solubilize the collagen contained therein. A catalyst which promotes the reconstitution of collagen fibers is then added to the extract, which is composed of a minimum quantity of collagen fibrils and which is free of nonfibrillar collagen precipitate. Following a maturation process, the reconstituted organized structures are separated from the aqueous medium by centrifuging, washed, aseptized, conditioned, lyophilized, and sterilized. The collagen fibers appear in the isolated state in the form of a web ready for use.

Miyata et al., Japanese patent application 4-227265, published August 17, 1992, discloses a composition comprising atelopeptide collagen linked to a polyepoxy compound. The composition is injected into the body to obtain sustained skin-lifting effects.

Zalipsky et al., Poly(Ethylene Glycol) Chemistry: Biotechnical and Biomedical Applications, Plenum Press: New York, NY (1992) 347-355 and Biotech. & Appl. Biochem. (1992) 15:110-114, measured rates of reactivity for various types of functionally activated polyethylene glycols.

Commonly owned U.S. Patent 5,162,430, issued November 10, 1992 to Rhee et al., disclosed collagen-synthetic polymer conjugates, methods of covalently binding collagen to synthetic hydrophilic polymers, and methods of covalently binding biologically active agents to collagen-synthetic polymer conjugates. Commonly owned, allowed U.S. application Serial No. 07 / 922,541, filed July 30, 1992, disclosed various activated forms of polyethylene glycol and various linkages which can be used to produce collagen-synthetic polymer conjugates having a range of physical and chemical properties. Commonly owned U.S. application serial No. 07 / 984,933, filed December 2, 1992, disclosed methods for coating implants with collagen-synthetic polymer conjugates. Commonly owned, allowed U.S. application serial No. 07 / 985,680, filed December 2, 1992 disclosed methods for making tubes comprising collagen-synthetic polymer conjugates.

All publications mentioned herein are incorporated herein by reference to describe and disclose the subject matter for which it is cited.

We now disclose improved collagen-synthetic polymer conjugates comprising collagens covalently bound to synthetic hydrophilic polymers, and methods for producing these conjugates. These improved collagen-synthetic polymer conjugates have superior physical characteristics due to the relatively homogeneous collagen fiber size population.

## Definitions

It must be noted that, as used in this specification and the appended claims, the singular forms "a", "an", and "the" include plural referrents unless the context clearly dictates otherwise. For example, reference to "a conjugate" includes one or more conjugate molecules, reference to "an article" includes one or more different types of articles known to those skilled in the art and reference to "the collagen" includes mixtures of different types of collagens and so forth.

Specific terminology of particular importance to the description of the present invention is defined below:

The term "aqueous mixture" of collagen includes liquid solutions, suspensions, dispersions, colloids, and the like, containing collagen and water.

The term "atelopeptide collagen" refers to collagens which have been chemically treated or otherwise processed to remove the telopeptide regions, which are known to be responsible for causing an immune response in humans to collagens from other animal, such as bovine, sources.

The term "available lysine residue" as used herein refers to lysine side chains exposed on the outer surface of collagen molecules, which have free amino groups capable of reacting with activated polymeric glycols. The number of available lysine residues may be determined by reaction with sodium 2,4,6-

trinitrobenzenesulfonate (TNBS).

The term "biologically active molecules" is used to describe molecules such as growth factors, cytokines, and active peptides (which may be either naturally occurring or synthetic) which aid in the healing or regrowth of normal tissue. The function of biologically active molecules such as cytokines and growth factors is two-fold: 1) they can incite local cells to produce new tissue, or 2) they can attract cells to the site in need of correction. As such, biologically active molecules serve to encourage "biological anchoring" of an implant within the host tissue. Biologically active molecules useful in conjunction with the collagen-synthetic polymer conjugates of the present invention include, but are not limited to, cytokines such as interferons (IFN), tumor necrosis factors (TNF), interleukins, colony stimulating factors (CSFs), and growth factors such as osteogenic factor extract (OFE), epidermal growth factor (EGF), transforming growth factor (TGF) alpha, TGF-$\beta$ (including any combination of TGF-$\beta$s), TGF-$\beta$1, TGF-$\beta$2, platelet derived growth factor (PDGF-AA, PDGF-AB, PDGF-BB), acidic fibroblast growth factor (FGF), basic FGF, connective tissue activating peptides (CTAP), $\beta$-thromboglobulin, insulin-like growth factors, erythropoietin (EPO), and nerve growth factor (NGF).

The term "chemically conjugated" as used herein means attached through a covalent chemical bond. In the practice of the invention, a hydrophilic synthetic polymer and a collagen molecule may be covalently conjugated directly to each other by means of a functionally active group on the synthetic hydrophilic polymer, or the collagen and synthetic polymer may be covalently conjugated using a linking radical, so that the hydrophilic synthetic polymer and collagen are each bound to the radical, but not directly to each other.

The term "collagen" as used herein refers to all forms of collagen which can be used as starting materials, including those which have been recombinantly produced, extracted from naturally occurring sources (such as bovine corium or human placenta), processed, or otherwise modified.

The term "collagen suspension" refers to a suspension of collagen fibers in an aqueous carrier, such as water or phosphate-buffered saline (PBS).

The term "collagen-synthetic polymer" refers to collagen chemically conjugated to a synthetic hydrophilic polymer, within the meaning of this invention. For example, "PEG-collagen" denotes a composition of the invention wherein molecules of collagen are covalently conjugated to molecules of polyethylene glycol (PEG).

"Crosslinked collagen" refers to a collagen composition in which collagen molecules are linked by covalent bonds with multifunctionally activated synthetic hydrophilic polymers, such as difunctionally activated polyethylene glycol.

The term "dehydrated" means that the material is air-dried or lyophilized to remove substantially all unbound water.

The term "difunctionally activated" refers to synthetic hydrophilic polymers which have been chemically derivatized so as to have two functional groups capable of reacting with available lysine residues on collagen molecules. The two functionally activate groups on a difunctionally activated synthetic hydrophilic polymer are generally located one at each end of the polymer chain. Each functionally activated group on a difunctionally activated synthetic hydrophilic polymer molecule is capable of covalently binding with a collagen molecule, thereby effecting crosslinking between the collagen molecules.

The term "effective amount" refers to the amount of a composition required in order to obtain the effect desired. Thus, a "tissue growth-promoting amount" of a composition containing a biologically active molecule refers to the amount of biologically active molecule needed in order to stimulate tissue growth to a detectable degree. Tissue, in this context, includes any tissue of the body. The actual amount which is determined to be an effective amount will vary depending on factors such as the size, condition, sex, and age of the patient, and can be more readily determined by the caregiver.

The term "fiber size" refers generally to the diameter of a discrete collagen fiber, as measured using the AccuSizer® 770 Optical Particle Sizer, Software C770, Version 2.0 (manufactured by Particle Sizing Systems, Inc., Santa Barbara, California), or equivalent particle analysis system.

The terms "fiber size population", "fiber size distribution", and "fiber size population distribution" refer to the range of fiber sizes found in a collagen sample as a whole.

The term "fibrillar collagen" refers to collagens in which the triple helical molecules aggregate to form thick fibers due to intermolecular charge interactions, such that a composition containing fibrillar collagen will be more or less opaque. Fibrillar collagen generally contains thick collagen fibers having a mean fiber size of at least 10 - 12 microns.

The term "functionally activated" refers to synthetic hydrophilic polymers which have been chemically derivatized so as to have one or more functional group capable of reacting with available lysine residues on collagen molecules at various locations along the polymer chain.

The terms "implant" and "solid implant" refer to any semi-solid or solid object which is intended for insertion and long- or short-term use within the body.

The term "*in situ*" as used herein means at the site of administration.

The term "*in situ* crosslinking" as used herein refers to crosslinking of a collagen implant to a patient's own collagen using multifunctionally activated synthetic polymers, wherein one functionally activated end of the synthetic polymer is covalently conjugated to a collagen molecule in the collagen implant, and the other functionally activated end of the polymer is free to covalently bind to collagen molecules within the patient's own tissue.

The term "mean fiber size" refers to the number-weighted mean fiber size which is determined by dividing the additive sizes of the collagen fibers measured by the number of collagen fibers measured.

The term "microfibrillar collagen" refers to collagens in which the triple helical molecules aggregate to form thin fibers, such that a composition containing microfibrillar collagen will be more or less translucent. Microfibrillar collagen generally contains thin collagen fibers having a mean fiber size of about 7.0 microns or less, and a fiber size distribution wherein at least 80% of the fibers have a fiber size of about 10 microns or less.

The term "molecular weight" as used herein refers to the weight average molecular weight of a number of molecules in any given sample, as commonly used in the art. Thus, a sample of PEG 2000 might contain a statistical mixture of polymer molecules ranging in weight from, for example, 1500 to 2500, with one molecule differing slightly from the next over a range. Specification of a range of molecular weight indicates that the average molecular weight may be any value between the limits specified, and may include molecules outside those limits. Thus, a molecular weight range of about 800 to about 20,000 indicates an average molecular weight of at least about 800, ranging up to about 20,000.

The term "monofunctionally activated" refers to synthetic hydrophilic polymers which have been chemically derivatized so as to have one functional group capable of reacting with an available lysine residue on a collagen molecule. The functionally activate group on a monofunctionally activated synthetic hydrophilic polymer is generally located at one end of the polymer chain. Because they can only bind to one collagen molecule at a time, monofunctionally activated synthetic hydrophilic polymers are not capable of effecting crosslinking between collagen molecules.

The term "multifunctionally activated" refers to synthetic hydrophilic polymers which have been chemically derivatized so as to have two or more functional groups capable of reacting with available lysine residues on collagen molecules at various locations along the polymer chain. Each functionally activate group on a multifunctionally activated synthetic hydrophilic polymer molecule is capable of covalently binding with a collagen molecule, thereby effecting crosslinking between the collagen molecules. Types of multifunctionally activated hydrophilic synthetic polymers include difunctionally activated, tetrafunctionally activated, and star-branched polymers.

The term "nonfibrillar collagen" refers to collagens in which the triple helical molecules do not aggregate to form thick fibers, such that a composition containing nonfibrillar collagen will be optically clear. "Nonfibrillar" collagen generally contains very small collagen fibers having a mean fiber size of about 6.5 microns or less, and a fiber size distribution wherein at least 80% of the fibers have a fiber size of about 8.0 microns or less.

The term "optically clear" as used herein refers to an article which transmits at least 90% of the visible light directed at it at a thickness of 1 mm.

The term "pharmaceutically acceptable fluid carrier" refers to fluid carriers for use in injectable or implantable formulations which are biocompatible (*i.e.*, do not invoke an adverse response when injected or otherwise implanted within the human body) and which may be either aqueous, such as water or PBS, or nonaqueous, such as a biocompatible oil.

The term "reprecipitated collagen" refers to collagens which have been prepared by acidification of fibrillar collagens, which are originally at approximately neutral pH (~ 6 - 9), then adjustment of the pH of the collagen to a pH wherein the collagen contains the desired fiber size distribution, *e.g.*, wherein at least 80% of the collagen fibers have a fiber size of about 10 microns or less.

The term "sufficient amount" as used herein is applied to the amount of acid, base, or salt which must be added to the collagen composition in order to achieve the desired pH and/or fiber size.

The terms "synthetic hydrophilic polymer" or "synthetic polymer" refer to polymers which have been synthetically produced and which are hydrophilic, but not necessarily water-soluble. Examples of synthetic hydrophilic polymers which can be used in the practice of the present invention are polyethylene glycol (PEG), polyoxyethylene, polymethylene glycol, polytrimethylene glycols, polyvinylpyrrolidones, polyoxyethylene-polyoxypropylene block polymers and copolymers, and derivatives thereof. Naturally occurring polymers such as proteins, starch, cellulose, heparin, hyaluronic acid, and derivatives thereof are expressly

excluded from the scope of this definition.

The term "translucent" as used herein refers to an article which transmits at least 50% of the visible light directed at it at a thickness of 1 mm.

The terms "treat" and "treatment" as used herein refer to replacement, augmentation, repair, prevention, or alleviation of defects related to soft and/or hard tissue. Additionally, "treat" and "treatment" also refer to the prevention, maintenance, or alleviation of disorders or disease using a biologically active molecule coupled to or mixed with the conjugates of the invention.

Except as otherwise defined above, all technical and scientific terms used herein have the same meaning as commonly understood by one of ordinary skill in the art to which this invention belongs. Although any methods and materials similar or equivalent to those described herein may be useful in the practice or testing of the present invention, only the preferred methods and materials are described below. It is not intended that the invention be limited to these preferred embodiments, however. The invention is intended to have the scope defined by the attached claims.

## Summary of the Invention

The present invention discloses conjugates comprising collagens having controlled fiber size distributions covalently bound to synthetic hydrophilic polymers, as well as implants comprising such improved collagen-synthetic polymer conjugates. Specifically, the invention discloses collagen-synthetic polymer conjugates wherein at least 80% of the collagen fibers have a fiber size of 10 microns or less. The invention also discloses collagen-synthetic polymer conjugates wherein the mean collagen fiber size is between about 5.0 and about 8.0 microns.

There are a number of methods for modifying the fiber size of collagen. These include reprecipitation of fibrillar collagen and/or addition of salt to a fibrillar collagen composition and/or temperature modification. In a general method for preparing an improved collagen-synthetic polymer conjugate of the invention, a collagen suspension comprising collagen fibers, wherein at least 80% of the collagen fibers have a fiber size of about 10 microns or less, is mixed with a solution of a synthetic hydrophilic polymer and allowed to incubate for a sufficient time to allow covalent binding to occur between the collagen and the synthetic polymer. In another general method, a suspension of reprecipitated collagen having a pH of about 6 or less is mixed with a solution of a synthetic hydrophilic polymer and allowed to incubate for a sufficient time to allow covalent binding to occur between the collagen and the synthetic polymer.

In accordance with a preferred method for preparing an improved collagen-synthetic polymer conjugate of the invention, a suspension of fibrillar collagen having a starting pH within the range of between about 6 and about 9 is adjusted to have a pH of about 5 or less. The collagen suspension is allowed to incubate for a sufficient time to effect fiber disassembly of the collagen, then the pH of the collagen suspension is readjusted to between about 5 and about 6. The collagen is allowed to incubate for a sufficient time to achieve the desired fiber size, and then adjusted to a pH within the range of between about 6 and about 9. The collagen is then immediately mixed with a solution comprising a synthetic hydrophilic polymer and allowed to incubate for a sufficient time to produce an improved collagen-synthetic polymer conjugate.

According to another preferred method of the invention, a suspension of fibrillar collagen having a starting pH within the range of between about 6 and about 9 is adjusted to have a pH of about 5 or less. The collagen suspension is allowed to incubate for a sufficient time to effect fiber disassembly of the collagen, then the pH of the collagen suspension is readjusted to about 6 or less. The collagen is allowed to incubate for a sufficient time to achieve the desired fiber size, then mixed with a solution comprising a synthetic hydrophilic polymer and allowed to incubate for a sufficient time to produce an improved collagen-synthetic polymer conjugate of the invention.

In accordance with a preferred method for preparing optically clear nonfibrillar collagen-synthetic polymer conjugates, a suspension of fibrillar collagen having a starting pH within the range of between about 6 and about 9 is adjusted to have a pH of about 5 or less. The collagen suspension is allowed to incubate for a sufficient time at a temperature within the range of between about 0°C and 8°C to produce substantially nonfibrillar collagen. The pH of the nonfibrillar collagen is then adjusted to within the range of between about 6 and about 9, then immediately mixed with a solution comprising a synthetic hydrophilic polymer and allowed to incubate for a sufficient time at a temperature within the range of between about 0°C and about 8°C to produce an optically clear nonfibrillar collagen-synthetic polymer conjugate.

In yet another method for preparing improved collagen-synthetic polymer conjugates of the invention, salt is added to a suspension of fibrillar collagen having a pH within the range of between about 6 and about 9 in an amount sufficient to reduce the fiber size of the collagen such that at least 80% of the collagen fibers have a fiber size of about 10 microns or less. The collagen is then mixed with a solution of a

synthetic hydrophilic polymer and allowed to incubate for a sufficient time to allow covalent binding to occur between the collagen and the synthetic polymer.

The improved collagen-synthetic polymer conjugates of the invention can be used to produce formed implants for use in a variety of medical application.

The present invention provides conjugates comprising collagen having controlled fiber size distributions covalently bound to hydrophilic synthetic polymers, and preferred methods for preparing the conjugates, including by reprecipitating fibrillar collagen and crosslinking the resulting collagen with synthetic hydrophilic polymers once the desired fiber size range has been achieved.

## Brief Description of the Drawings

Figure 1 shows differential scanning calorimetry (DSC) results for a collagen composition at pH 3.0.

Figure 2 shows DSC results for the collagen composition of Figure 1, adjusted to pH 4.0.

Figure 3 shows DSC results for the collagen composition of Figure 1, adjusted to pH 5.0.

Figure 4 shows DSC results for the collagen composition of Figure 1, adjusted to pH 5.5.

Figure 5 shows DSC results for the collagen composition of Figure 1, adjusted to pH 6.3.

Figure 6 shows DSC results for the collagen composition of Figure 1, adjusted to pH 7.2.

Figure 7 shows DSC results for the collagen composition of Figure 1, adjusted to pH 7.2, with 0.26 M NaCl added.

Figure 8 shows the fiber size population distribution of a collagen composition at pH 3.0.

Figure 9 shows the fiber size population distribution of the collagen composition of Figure 7, adjusted to pH 4.0.

Figure 10 shows the fiber size population distribution of the collagen composition of Figure 7, adjusted to pH 5.0.

Figure 11 shows the fiber size population distribution of the collagen composition of Figure 7, adjusted to pH 5.5.

Figure 12 shows the fiber size population distribution of the collagen composition of Figure 7, adjusted to pH 6.3.

Figure 13 shows the fiber size population distribution of the collagen composition of Figure 7, adjusted to pH 7.3.

Figure 14 shows the fiber size population distribution of the collagen composition of Figure 7, adjusted to pH of 7.3, with 0.26 M NaCl added.

## Detailed Description of Preferred Embodiments of the Invention

The present invention discloses conjugates comprising collagens having controlled fiber size distributions covalently bound to synthetic hydrophilic polymers, as well as implants comprising such improved collagen-synthetic polymer conjugates and methods for preparing said conjugates. Specifically, the invention discloses collagen-synthetic polymer conjugates wherein at least 80% of the collagen fibers have a fiber size of 10 microns or less. Collagen-synthetic polymer conjugates having a mean collagen fiber size between about 5.0 and about 8.0 microns are also disclosed.

It has been discovered that, due to mechanical and physical considerations, collagen suspensions having homogeneous fiber size populations (*i.e.*, a narrow range of fiber sizes) and, particularly, homogeneous populations of small fibers, can be crosslinked with synthetic hydrophilic polymers more efficiently and completely than collagen suspensions having heterogeneous fiber size populations (*i.e.*, a wide range of fiber sizes, which includes very large fibers). However, commercially available, noncrosslinked fibrillar collagen compositions, such as Zyderm® I Collagen Implant or Zyderm II Collagen Implant (both available from Collagen Corporation, Palo Alto, California), tend to contain a broad range of fiber sizes, including very large fibers. Therefore, the use of commercially available fibrillar collagens to prepare covalently crosslinked collagen-synthetic polymer conjugates results in crosslinking which is not as efficient, complete, or strong as would ideally be desired.

A number of these commercially available fibrillar collagen compositions can, however, be used as starting materials to prepare collagen compositions having relatively homogeneous, controlled fiber size populations and, in particular, homogeneous populations of small fibers. A number of methods can be used to control the fiber size of fibrillar collagen, including among them, reprecipitation of fibrillar collagen and/or addition of salt to a fibrillar collagen composition. We have found that reprecipitation of fibrillar collagen is a preferred method to prepare the improved collagen compositions of the invention, providing collagen compositions having relatively homogeneous, controlled fiber size populations. Furthermore, fibrillogenesis

(fiber formation) can be controlled such that materials are produced having a majority of fibers within the desired fiber size population to form the compositions of the invention, that is, at least 80% of the collagen fibers having a fiber size of about 10 microns or less. Once the desired fiber size population is achieved, the fiber size can be "locked in" by chemically crosslinking the collagen, such as by addition of a functionally activated polymeric glycol. The fiber size population selected will determine the physical properties of the resulting crosslinked collagen composition.

Because of the relatively uniform fiber size population of the collagen in the improved collagen-polymer conjugates, production of these crosslinked materials is more reproducible than production of previously described collagen-polymer conjugates. Furthermore, the materials themselves are more predictable in their physical and chemical properties and, therefore, more similar to synthetic biomaterials than previously described collagen-synthetic polymer conjugates. Because more complete crosslinking can be achieved due to the homogeneity of the fiber size population, it is expected that the crosslinked collagen formulations of the invention will be more persistent than previous collagen-synthetic polymer formulations. Because of the tighter crosslinking able to be achieved due to the , it is further believed that the improved crosslinked collagen formulations of the present invention will be even less immunogenic than previously disclosed collagen-synthetic polymer formulations.

In order to produce the improved collagen-synthetic polymer conjugates of the invention, it is necessary to prepare collagen having a controlled fiber size distribution and then covalently bind the collagen to a synthetic hydrophilic polymer, such as a functionally activated polymeric glycol. In accordance with one preferred method, (a) a functionally activated hydrophilic synthetic polymer is prepared or otherwise provided, (b) a collagen composition having the desired fiber size distribution is prepared or otherwise provided, and (c) the activated synthetic polymer is allowed to react with the collagen, resulting in covalent binding of the synthetic polymer to the collagen.

## Activation of Polyethylene Glycol (PEG)

A critical step in forming the improved collagen-synthetic polymer conjugates of the invention involves functionalization, or activation, of the synthetic hydrophilic polymer. The synthetic polymers useful in the present invention are hydrophilic, have at least one and, preferably, two or more functional groups capable of covalently bonding with the lysine residues on a collagen molecule, and are highly pure or purified to a highly pure state such that the polymer is or is treated to become pharmaceutically pure so that it may be injected into a human patient. Most hydrophilic synthetic polymers can be rendered water-soluble by incorporating a sufficient number of oxygen (or, less frequently, nitrogen) atoms available for forming hydrogen bonds in aqueous solutions. Preferred synthetic polymers are hydrophilic, but not necessarily water-soluble.

All suitable synthetic polymers will be nontoxic, noninflammatory, and nonimmunogenic when administered subcutaneously, and will preferably be essentially nondegradable *in vivo* over a period of at least several months. The hydrophilic synthetic polymer may increase the hydrophilicity of the conjugate, but does not render it water-soluble. The synthetic polymers can be linear or multiply branched, but are typically not substantially crosslinked.

Although different synthetic hydrophilic synthetic polymers can be used in connection with forming the conjugates of the invention, the synthetic polymer must be biocompatible, hydrophilic, but relatively insoluble in water, and is preferably one or more forms of derivatized polymeric glycol, preferablly, polyethylene glycol (PEG), due to its known biocompatibility. Various forms of derivatized PEG are extensively used in the modification of biologically active molecules because PEG can be formulated to have a wide range of solubilities and because it lacks toxicity, antigenicity, immunogenicity, and does not typically interfere with the enzymatic activities and/or conformations of peptides. Furthermore, PEG is generally non-biodegradable and is easily excreted from most living organisms including humans.

Multifunctionally activated synthetic polymers are most preferred for use in the present invention, with difunctionally activated polymers being most preferred. Multifunctionally activated polymeric glycols preferably have an average molecular weight between about 3000 and 100,000. Difunctionally activated polymeric glycols preferably have an average molecular weight of between about 400 to about 40,000, most preferably about 3000 to about 10,000. Although monofunctionally activated polymers may be used in the practice of the invention, they are generally not preferred. Because monofunctionally activated synthetic polymers have only one activated functional group, they are not capable of forming a crosslinked network among collagen molecules.

Multifunctionally activated synthetic polymers can be prepared using various techniques known in the art which provide functionally groups at various locations along the polymer. Difunctionally activated

polymeric glycols typically are prepared by constructing reactive hydroxy groups at the ends of the polymer. Multifunctionally activated synthetic polymers are capable of crosslinking the compositions of the invention, and may further be used to attach biologically active molecules to the collagen-synthetic polymer conjugate.

Various functionalized polyethylene glycols have been used effectively in fields such as protein modification (see Abuchowski et al., Enzymes as Drugs, John Wiley & Sons: New York, NY (1981) pp. 367-383; and Dreborg et al., Crit. Rev. Therap. Drug Carrier Syst. (1990) 6:315 ), peptide chemistry (see Mutter et al., The Peptides, Academic: New York, NY 2:285-332; and Zalipsky et al., Int. J. Peptide Protein Res. - (1987) 30:740), and the synthesis of polymeric drugs (see Zalipsky et al., Eur. Polym. J. (1983) 19:1177; and Ouchi et al., J. Macromol. Sci. -Chem. (1987) A24:1011). Various types of conjugates formed by the binding of functionally activated polyethylene glycol with specific pharmaceutically active proteins have been disclosed and found to have useful medical applications in part due to the stability of such conjugates with respect to proteolytic digestion, reduced immunogenicity, and longer half-lives within living organisms.

One form of polyethylene glycol is monomethoxy-polyethylene glycol (mPEG), which can be activated by the addition of a compound such as cyanuric chloride, then coupled to a protein (see Abuchowski et al., J. Biol. Chem. (1977) 252:3578). Although such methods of activating polyethylene glycol can be used in connection with the present invention, they are not preferred in that the cyanuric chloride is relatively toxic and must be completely removed from any resulting product in order to provide a pharmaceutically acceptable composition.

Activated forms of PEG can be made from reactants which can be purchased commercially. One form of activated PEG which has been found to be particularly useful in connection with the present invention is PEG-succinate-N-hydroxysuccinimide ester (SS-PEG) (see Abuchowski et al., Cancer Biochem. Biphys. - (1984) 7:175). Activated forms of PEG such as SS-PEG react with proteins under relatively mild conditions and produce conjugates without destroying the specific biological activity and specificity of the protein attached to the PEG. However, when such activated PEGs are reacted with proteins, they react and form linkages by means of ester bonds. Although ester linkages can be used in connection with the present invention, they are not particularly preferred for use in formed implants intended for long-term use within the human body in that they undergo hydrolysis when subjected to physiological conditions over extended periods of time (see Dreborg et al., Crit. Rev. Therap. Drug Carrier Syst. (1990) 6:315; and Ulbrich et al., J. Makromol. Chem. (1986) 187:1131).

It is possible to link PEG to proteins via urethane linkages, thereby providing a more stable attachment which is more resistant to hydrolytic digestion than the ester linkages (see Zalipsky et al., Polymeric Drug and Drug Delivery Systems, Chapter 10, "Succinimidyl Carbonates of Polyethylene Glycol" (1991)). The stability of urethane linkages has been demonstrated under physiological conditions (see Veronese et al., Appl. Biochem. Biotechnol. (1985) 11:141; and Larwood et al., J. Labelled Compounds Radiopharm. (1984) 21:603). Another means of attaching the PEG to a protein can be by means of a carbamate linkage (see Beauchamp et al., Anal. Biochem. (1983) 131:25; and Bergeret al., Blood (1988) 71:1641). The carbamate linkage is created by the use of carbonyldiimidazole-activated PEG. Although such linkages have advantages, the reactions are relatively slow and may take 2 to 3 days to complete.

The various means of activating PEG described above and publications cited in connection with the activation means are described in connection with linking PEG to specific biologically active proteins and not inert, biologically inactive, natural polymers such as collagen. (See Polymeric Drug and Drug Delivery Systems, Chapter 10, "Succinimidyl Carbonates of Polyethylene Glycol" (1991).) Such activated PEG compounds can be used in the preparation of covalently crosslinked conjugates of various collagens which can be used in the preparation of a variety of formed implants for use in medical applications.

Specific Forms of Activated PEG

For use in the present invention, polyethylene glycol is modified in order to provide activated groups on one or, preferably, two or more sites along the length of the PEG molecule, so that covalent binding can occur between the PEG and the free amino groups on a collagen molecule. Some specific activated forms of PEG are shown structurally below, as are generalized reaction products obtained by reacting activated forms of PEG with collagen. In Formulas 1 - 7, the term COL represents collagen. The term PEG represents polymers having the repeating structure $(CH_2CH_2OCH_2CH_2)_n$.

The first activated PEG is difunctionally activated PEG succinimidyl glutarate, referred to herein as (SG-PEG). The structural formula of this molecule and the reaction product obtained by reacting it with a collagen are shown in Formula 1.

SG-PEG: Difunctionally Activated PEG Succinimidyl Glutarate

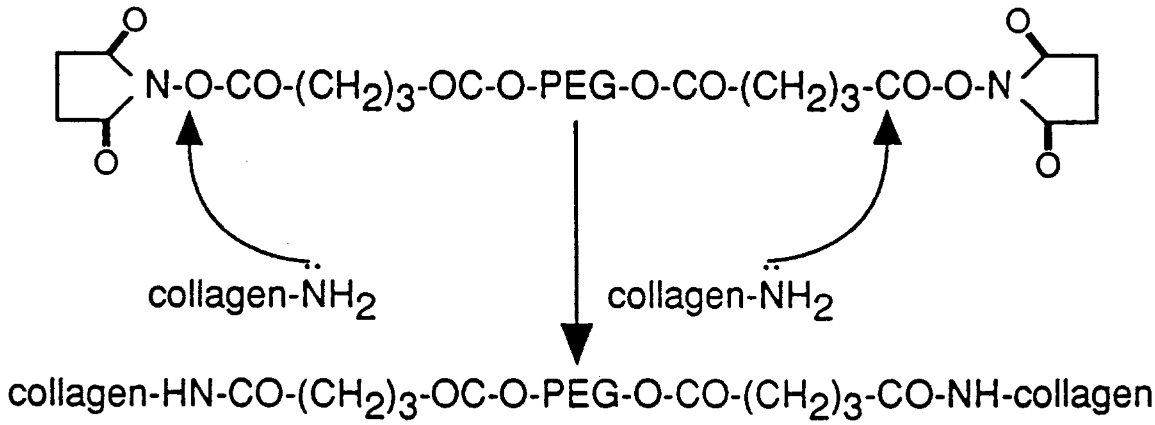

FORMULA 1

Another difunctionally activated form of PEG is referred to as PEG succinimidyl (S-PEG). The structural formula for this compound and the reaction product obtained by reacting it with collagen is shown in Formula 2. In any general structural formula for the compounds, the subscript 3 is replaced with an "n". In the embodiment shown in Formula 1, n = 3, in that there are three repeating $CH_2$ groups on either side of the PEG. The structure in Formula 2 results in a conjugate which includes an "ether" linkage which is not subject to hydrolysis. This is distinct from the conjugate shown in Formula 1, wherein an ester linkage is provided. The ester linkage is subject to hydrolysis under physiological conditions.

S-PEG, n = 3: Difunctionally Activated PEG Succinimidyl

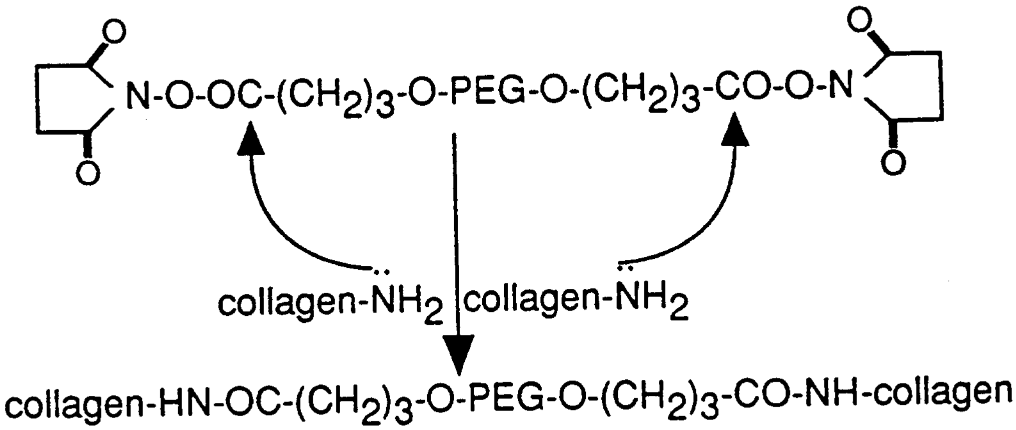

FORMULA 2

Yet another difunctionally activated form of polyethylene glycol, wherein n = 2, is shown in Formula 3, as is the conjugate formed by reacting the activated PEG with collagen.

S-PEG, n = 2: Difunctionally Activated PEG Succinimidyl

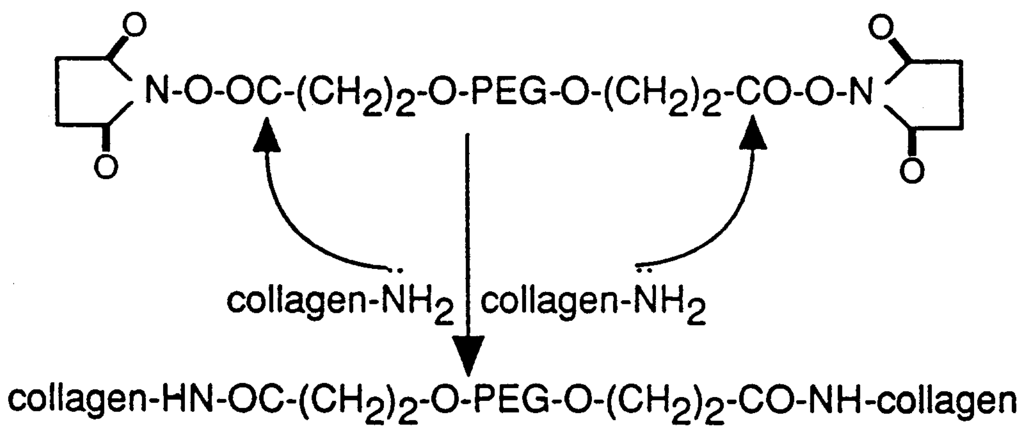

FORMULA 3

Another preferred embodiment of the invention similar to the compounds of Formulas 2 and 3 is provided when n = 1. The structural formula and resulting collagen-synthetic polymer conjugate are shown in Formula 4. It is noted that this conjugate includes both an ether and a peptide linkage. These linkages are stable under physiological conditions.

S-PEG, n = 1: Difunctionally Activated PEG Succinimidyl

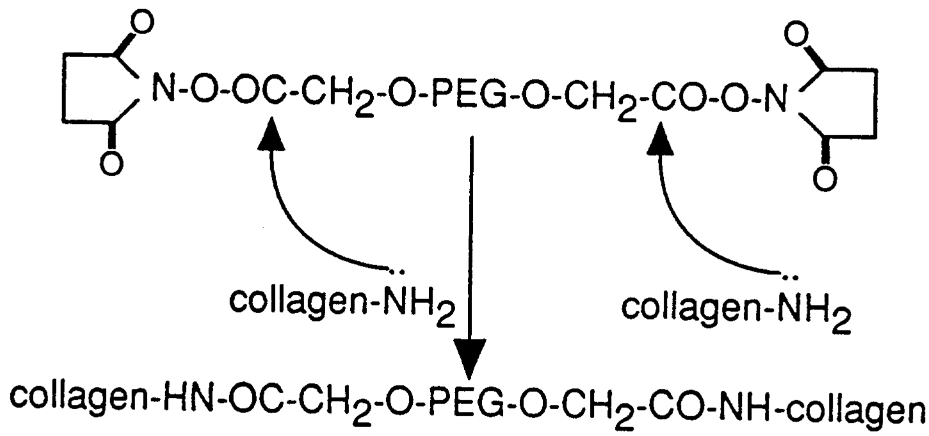

FORMULA 4

Yet another difunctionally activated form of PEG is provided when n = 0. This compound is referred to as PEG succinimidyl carbonate (SC-PEG). The structural formula of this compound and the conjugate formed by reacting SC-PEG with collagen is shown in Formula 5.

SC-PEG, n = 0; Difunctionally Activated PEG Succinimidyl Carbonate

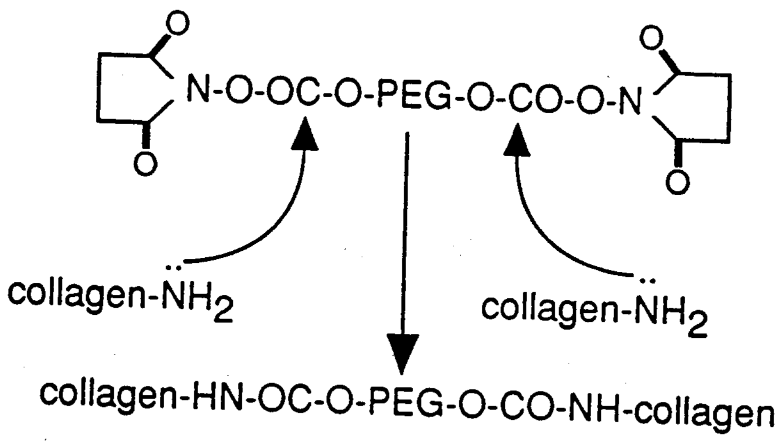

FORMULA 5

All of the activated polyethylene glycol derivatives depicted in Formulas 1 - 5 involve the inclusion of the succinimidyl group. However, different activating groups can be attached at sites along the length of the PEG molecule. For example, PEG can be derivatized to form difunctionally activated PEG propion aldehyde (A-PEG), which is shown in Formula 6, as is the conjugate formed by the reaction of A-PEG with collagen. The linkage shown in Formula 6 is referred to as a $-(CH_2)_n-NH-$ linkage, where n = 1 - 10.

A-PEG: Difunctionally Activated PEG Propion Aldehyde

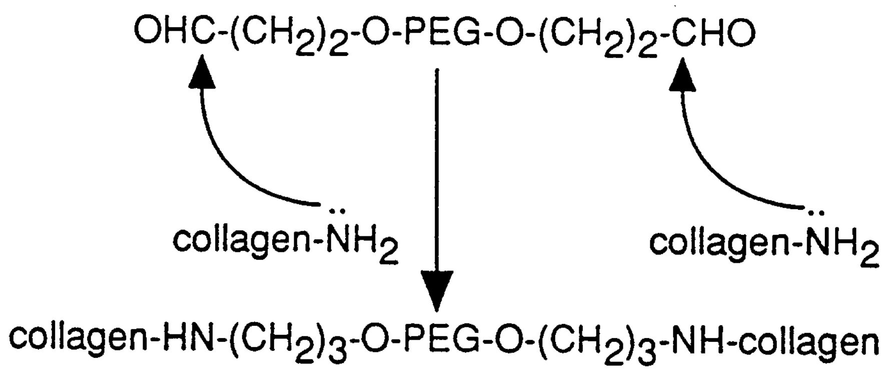

FORMULA 6

Yet another form of activated polyethylene glycol is difunctionally activated PEG glycidyl ether (E-PEG), which is shown in Formula 7, as is the conjugate formed by reacting such with collagen.

E-PEG: Difunctionally Activated PEG Glycidyl Ether

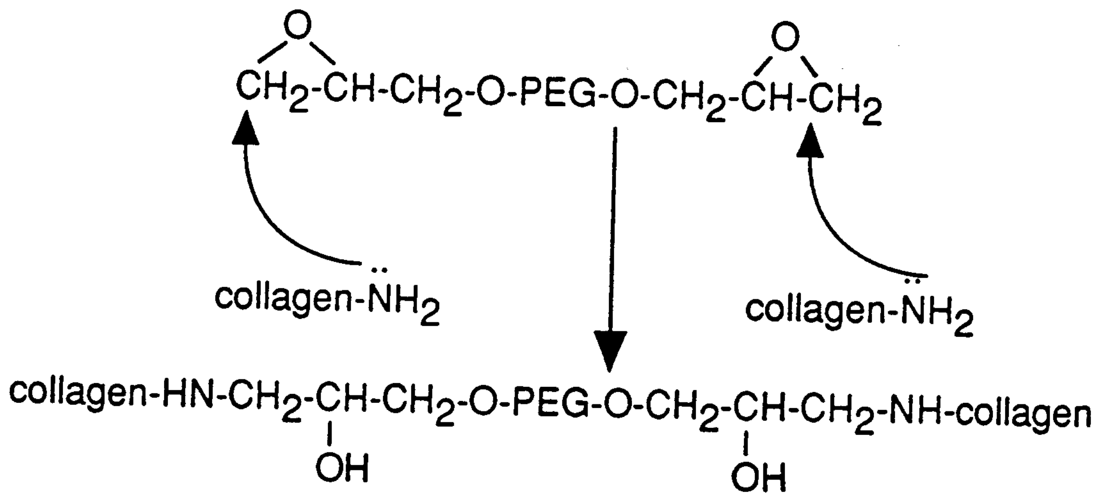

FORMULA 7

Many of the activated forms of polyethylene glycol described above are now available commercially from Shearwater Polymers, Huntsville, Alabama. The various activated forms of polyethylene glycol and various linkages which can be used to produce collagen-synthetic polymer conjugates having a range of physical and chemical properties are described in further detail in copending, allowed U.S. application Serial No. 07 / 922,541, filed July 2, 1992.

The specific form of functionally activated synthetic hydrophilic polymer used depends on the desired end use application of the implant prepared from the collagen-synthetic polymer conjugate. The type of linkage required between the collagen and the functionally activated polyethylene glycol will depend upon whether the implant is intended for long- or short-term implantation within the body of the patient. In general, functionally activated polyethylene glycols which result in ether or ester linkages are preferred for implants intended for long-term use because these linkages tend to be more resistant to hydrolysis than ester linkages. Polyethylene glycols which result in the weaker ester linkage may be used in short-term implants. In fact, ester linkages are preferred for implants intended to provide localized drug delivery. The covalently bound or admixed drug is released from the collagen-synthetic polymer matrix as the ester bonds are hydrolyzed.

Preparation of Collagen Having a Controlled Fiber Size Population

Collagen obtained from any source may be used to prepare the injectable collagen-synthetic polymer conjugates of the present invention. Collagen may be extracted and purified from human or other mammalian source, or may be recombinantly or otherwise produced. Collagen of any type may be used, including, but not limited to, types I, II, III, IV, or any combination thereof, although type I is generally preferred. Atelopeptide collagen is generally preferred over telopeptide-containing collagen because of its reduced immunogenicity. Collagens that have been previously crosslinked by radiation, heat, or other chemical crosslinking agents such as glutaraldehyde or carbodiimide, or treated in any such manner which would interfere with or inhibit fiber size adjustment of the collagen, are generally not preferred as starting materials to prepare the collagen-synthetic polymer conjugates of the invention. Collagen used to prepare

the collagen-synthetic conjugates of the invention should be in a pharmaceutically pure form such that it can be incorporated into a human body for the intended purpose without generating any significant immune response.

Fibrillar collagen prepared by methods known in the art or commercially available atelopeptide fibrillar collagen compositions, such as Zyderm® I Collagen (35 mg/ml collagen concentration) or Zyderm II Collagen (65 mg/ml collagen concentration), are the preferred starting materials to prepare the collagens of the present invention. The starting materials are generally at approximately neutral pH, *i.e.*, pH 6 - 9. The collagen concentration of the collagen suspension used to make the improved collagen-synthetic polymer conjugates of the invention will generally be within the range of about 10 to about 120 mg/ml, depending on the desired end use application. The collagen concentration of commercially available collagen compositions can be decreased prior to reprecipitation by mixing the collagen composition with an appropriate amount of sterile water or phosphate buffered saline (PBS). Conversely, to increase the collagen concentration, the collagen composition can be concentrated by centrifugation, then adjusted to the desired collagen concentration by mixing with an appropriate amount of sterile water or PBS.

Due to intermolecular charge interactions, native collagen molecules aggregate to form collagen fibers at approximately pH 5 and above. Therefore, to reprecipitate fibrillar (insoluble) collagen and control subsequent fibrillogenesis, it is first necessary to acidify the collagen to pH 5 or less, preferably, between about pH 3 and about pH 5, and then incubate the collagen for a sufficient time (generally, a minimum of one hour) to obtain nonfibrillar (soluble) collagen. Acidification of the collagen suspension can be effected by the addition of an appropriate amount of an acid such as hydrochloric acid, for example.

Following acidification, the collagen suspension is adjusted to the specific pH required to achieve the desired fiber size by the addition of a base such as a phosphate buffer ($Na_2HPO_4$ / $NaH_2PO_4$), an acetate buffer (NaAc / HAc), or sodium hydroxide. As the fiber size achieved is determined by the pH of the collagen suspension (*i.e.*, increases in pH result in the production of larger fibers), it is possible to select the fiber size population desired by controlling the pH of the collagen suspension. Therefore, if a homogeneous population consisting of small fibers is desired, it is necessary to maintain the pH of the collagen suspension between about 3 and about 5. Larger fibers will be generated as the pH is increased above 5.

Once the desired pH is attained, the collagen suspension should be allowed to incubate for a sufficient time (generally, a minimum of one hour) at the desired pH to allow fibrillogenesis to occur and the desired fiber size population to be achieved. The rate of fibrillogenesis can be controlled by controlling the temperature at which fibrillogenesis occurs. The rate of fibrillogenesis is directly correlated to temperature, *i.e.*, the lower the temperature, the slower the rate of fibrillogenesis. However, if fibrillogenesis is allowed to proceed for a long time (*i.e.*, about 7 - 9 hours) at low temperature, the resulting fibers formed will grow larger. Conversely, at elevated temperatures, fibrillogenesis occurs very rapidly, but the maximum fiber size achieved will be smaller than that achieved at low temperatures.

In another method for controlling the fiber size of collagen, a salt, such as, for example, sodium chloride, potassium chloride, sodium phosphate, or calcium chloride, can be added to the collagen composition. Addition of salt to the collagen suspension disrupts the fiber structure of the collagen by producing ions which affect the ability of the collagen molecules to aggregate to form thick fibers, resulting in smaller (thinner) fibers. Addition of salt can optionally be used in conjunction with reprecipitation of collagen; that is, salt can be added to the collagen suspension during readjustment of the pH to neutral in order to the collagen fibers from growing any larger.

## Preparation of Improved Collagen - Synthetic Polymer Conjugates

To effect crosslinking with a functionally activated polyethylene glycol (PEG), the collagen suspension may either be maintained at about pH 6 or less; or, may be mixed with a solution of activated PEG at basic pH (*i.e.*, pH 8 - 9.5); or, may be neutralized to between about pH 6 and about pH 9 (preferably beween about pH 6.5 and pH 8, and, most preferably, approximately pH 7), then immediately (within 5 minutes or less of neutralizing the collagen suspension) mixed with a solution of activated PEG before further fibrillogenesis can occur.

A solution of functionally activated PEG in PBS is generally prepared just prior to mixing with the collagen suspension to minimize loss of activity due to hydrolysis. The concentration of the activated PEG solution required to prepare the collagen-synthetic polymer conjugates of the invention generally ranges from about 5 to about 100 milligrams of activated PEG per milliliter of PBS. The amount of activated PEG used will vary depending on the collagen concentration used, the type of activated PEG used (*e.g.*, S-PEG, SG-PEG, etc.), and the degree of crosslinking desired, which will depend on the desired end use application of the material.

The rate of the crosslinking reaction between PEG and collagen is determined by the type of PEG used and the pH and temperature of the reaction mixture. Reactivity of several types of PEG has been shown to increase with increases in pH. Zalipsky et al. (Biotech. & Appl. Biochem. (1992) 15:110-114) measured first order rate constants for hydrolysis ($K_h$) and aminolysis ($K_{am}$) of SC-PEG and SS-PEG. First order rate constants for hydrolysis ($K_h$) of difunctionally activated SG-PEG (MW 3400) are shown in Example 3 and follow the same trend demonstrated by Zalipsky et al. for SC-PEG and SS-PEG. Hydrolysis (reaction with water) and aminolysis (reaction with protein) are competing reactions involving nucleophilic substitution. Because a nucleophile (such as an OH- group or an amino group of a collagen molecule) attacks the carbon atom using a pair of its own electrons, the pH of the reaction solution has an important effect on the rate of the nucleophilic substitution reaction. Basic solutions tend to drive the nucleophilic substitution reaction, resulting in faster rates of reactivity.

Therefore, if faster rates of reaction between the collagen and activated PEG are required for a specific application, it is first necessary to increase the pH of the collagen suspension to between about 6 to about 9 (preferably between about 6.5 to about 8, and, most preferably, approximately 7) prior to immediate reaction with activated PEG. Reaction with activated PEG must be performed immediately following reprecipitation to prevent further fibrillogenesis from occurring and to "lock in" the desired fiber size of the collagen. In an alternative embodiment, rather than neutralizing the pH of the collagen itself, the collagen suspension is mixed with a basic (pH 8 - 9.5) solution of a functionally activated polymer to neutralize the pH of the reaction mixture.

If slower reaction rates are acceptable, it is preferable to react the collagen with activated PEG while maintaining the pH at which the desired fiber size was obtained. Complete crosslinking will generally be achieved within 2 to 24 hours, depending on the collagen concentration used, the activated PEG concentration used, and the pH and temperature of the reaction mixture (*i.e.*, the lower the pH and/or the temperature, the longer the reaction time required to effect complete crosslinking).

In a general method for preparing an improved collagen-synthetic polymer conjugate of the invention, a collagen suspension comprising collagen fibers, wherein at least 80% of the collagen fibers have a fiber size of about 10 microns or less, is mixed with a solution of a synthetic hydrophilic polymer and allowed to incubate for a sufficient time to allow covalent binding to occur between the collagen and the synthetic polymer. In a related method, a suspension of reprecipitated collagen having a pH of about 6 or less is mixed with a solution of a synthetic hydrophilic polymer and allowed to incubate for a sufficient time to allow covalent binding to occur between the collagen and the synthetic polymer.

In one preferred method for preparing the improved collagen-synthetic polymer conjugates of the invention, a suspension of fibrillar collagen is adjusted to about pH 5 or less (preferably, between about pH 3 and about pH 5) and allowed to incubate for a sufficient time to effect fiber disassembly, readjusted to between about pH 5 and about pH 6 and allowed to incubate for a sufficient time to achieve the desired fiber size, then readjusted to between about pH 6 and about pH 9 (preferably, between about pH 6.5 and about pH 8, most preferably, pH 7) and immediately mixed with a solution of functionally activated PEG to effect crosslinking. Complete crosslinking of the collagen with the activated polyethylene glycol will generally be achieved within approximately 20 to 40 minutes at room temperature using this method.

In another preferred method, a suspension of fibrillar collagen is adjusted to about pH 5 or less (preferably, between about pH 3 and about pH 5) and allowed to incubate for a sufficient time to effect fiber disassembly, readjusted to between about pH 5 and about pH 6 and allowed to incubate for a sufficient time to achieve the desired fiber size, then mixed with a solution of functionally activated PEG to effect crosslinking. Complete crosslinking of the collagen with the activated polyethylene glycol will generally require a minimum of 2 hours at room temperature using this method.

If desired, nonfibrillar collagen-synthetic polymer conjugates can be produced by adjusting the pH of a fibrillar collagen starting material to less than 5 (preferably, between about 3 and about 5), incubating the material for several hours at 0 - 8°C, then adjusting the pH of the collagen to between about pH 6 and about pH 9 (preferably, between about pH 6.5 and about pH 8, most preferably, pH 7) and immediately mixing the collagen with a solution of activated PEG in an ice bath at 0 - 8°C to prevent fiber formation from occurring. Alternatively, rather than neutralizing the pH of the collagen solution itself, the collagen can be mixed with a solution of activated PEG having a pH within the range of about 8 to about 9.5 to neutralize the pH of the reaction mixture. Nonfibrillar collagen-synthetic polymer conjugates are optically clear and can be used to formulate a variety of ophthalmic devices.

Optical clarity/opacity is a function of collagen fiber size, *i.e.*, the larger the fibers, the more opaque the composition. Therefore, collagens containing a relatively homogeneous population of small fibers (*i.e.*, at least 80% of the collagen fibers have a fiber size of about 9.0 or, preferably, 8.5 microns, or less) will be more or less translucent. Collagen compositions wherein at least 80% of the collagen fibers have a fiber

size of about 8.0 microns or less will be optically clear.

In another method for preparing improved collagen-synthetic polymer conjugates of the invention, a salt such as sodium chloride is added to a suspension of fibrillar collagen having a pH within the range of between about 6 and about 9 in an amount sufficient to reduce the fiber size of the collagen such that at least 80% of the collagen fibers have a fiber size of about 10 microns or less. The collagen is then mixed with a solution of a synthetic hydrophilic polymer and allowed to incubate for a sufficient time to allow covalent binding to occur between the collagen and the synthetic polymer.

Methods of covalently binding collagen to PEG are described in further detail in commonly owned U.S. Patent No. 5,162,430, of Rhee et al., issued November 10, 1992.

## Use and Administration

The improved collagen-synthetic polymer conjugates of the invention can be cast into various shapes to produce a variety of formed implants for use in different therapeutic applications. For instance, the conjugates can be formed into the shape of a tube for use in both vascular and non-vascular lumens of the body. In a general method for forming tubes, a mandrel is dipped into a solution comprising a collagen and a functionally activated hydrophilic synthetic polymer, before substantial crosslinking has occurred. The collagen and synthetic polymer are then allowed to crosslink and form a gel. The collagen-synthetic polymer conjugate material is allowed to dry on the mandrel. Once dry, the resulting collagen-synthetic polymer tube can be easily pushed off the mandrel. The tubes can be stored dry and then rehydrated just prior to use by soaking in sterile water. Due to the hydrophilic nature of the synthetic polymer, the rehydrated tubes will expand to their original size.

In an alternative method for making improved collagen-synthetic polymer tubes, the collagen and activated synthetic polymer are extruded into a hollow cylindrical mold prior to crosslinking. The collagen and synthetic polymer are allowed to crosslink in the mold. The resulting tubes can be removed from the mold in hydrated form or allowed to dry while still in the mold.

The improved collagen-synthetic polymer tubes can be used to make vascular grafts or stents, or as replacements for any other damaged or defective nonvascular lumen within the body, such as in the reproductive or urological systems, for example, damaged fallopian tubes or ureters. Methods for making tubes comprising collagen-synthetic polymer conjugates are described in further detail in copending, allowed U.S. application serial No. 07 / 985,680, filed December 2, 1992.

The improved collagen-synthetic polymer conjugates can also be used to make other types of formed implants, for example, artificial organs or heart valves. In a general method for producing formed implants, the collagen and functionally activated synthetic polymer are mixed and cast into a mold having the desired size and shape before substantial crosslinking has occurred between the collagen and polymer. The collagen and synthetic polymer are allowed to incubate and crosslink within the mold. The covalently crosslinked material can be removed from the mold in either the hydrated or dehydrated state. The resulting formed implant can be conveniently stored in dry form, then rehydrated prior to implantation within the patient's body.

The improved collagen-synthetic polymer conjugates can also be used to coat implants for implantation within the body, including, without limitation, bone and joint prostheses, coiled platinum wires for treating aneurysms, breast implants, catheters, artificial organs, vascular grafts (such as Dacron® or Teflon® grafts) and stents, sutures, and artificial ligaments or tendons. To prepare a coated implant, collagen having a relatively homogeneous, controlled fiber size disrtribution and a functionally activated synthetic polymer are mixed. The implant is dipped into the collagen-synthetic polymer solution before substantial crosslinking has been achieved. If dipping is not possible, the collagen-polymer solution may be applied to the surface of the implant by any other suitable method, such as painting or brushing. The collagen and synthetic polymer are allowed to crosslink and dry on the surface of the implant. The coated implant can be implanted with the coating in dehydrated form. Upon implantation, the collagen-synthetic polymer coating will rehydrate and swell to provide a more intimate fit with the surrounding tissue than conventional coated implants. Methods for coating implants with collagen-synthetic polymer conjugates are described in further detail in copending U.S. application serial No. 07 / 984,933, filed December 2, 1992.

The improved collagen-synthetic polymer conjugates of the invention can also be formed into membranes for use in a variety of medical applications, such as adhesion prevention or guided tissue regeneration. Preparation of membranes is accomplished by casting a solution comprising collagen and a functionally activated synthetic polymer as a thin layer on the bottom of a flat sheet container, allowing the collagen and polymer to crosslink, then, optionally, lyophilizing the resulting thin membrane.

Because of their optical clarity, the nonfibrillar collagen-synthetic polymer conjugates prepared according to the present invention can be used to prepare a variety of devices for use in ophthalmic applications, such as corneal lenticules, corneal shields for drug delivery, or artificial corneas and lenses.

Fluid, injectable formulations can be prepared by admixing the collagen-synthetic polymer conjugates with appropriate amounts of pharmaceutically acceptable fluid carriers. Injectable formulations of the collagen-synthetic polymer conjugates generally have collagen concentrations within the range of about 10 to about 120 mg/ml and, preferably, about 20 to about 90 mg/ml, most preferably, about 30 to about 70 mg/ml. Injectable compositions may be crosslinked fully outside the body, or may be partially crosslinked outside the body, then further crosslinked *in situ* to the patient's own collagen to anchor the implant in place. The concept of *in situ* crosslinkable implants comprising collagen-synthetic polymer conjugates is discussed in greater detail in commonly owned U.S. Patent No. 5,162,430.

Biologically active molecules, such as growth factors and cytokines, can optionally be incorporated into formed implants and injectable formulations comprising the improved collagen-synthetic polymer conjugates of the invention. Injectable collagen-synthetic polymer formulations containing biologically active molecules can function as effective sustained release delivery systems for the active agents.

Implant coatings comprising the collagen-synthetic polymer conjugates might also include growth factors which promote fixation of the implant within the host tissue, such as one or more bone morphogenic proteins or osteogenic factors in the case of a bone prosthesis. Factors which prevent tissue ingrowth between organs may be incorporated into membranes for use in adhesion prevention.

Biologically active agents may be admixed with the collagen and functionally activated synthetic polymer before substantial crosslinking has occurred, resulting in a homogeneous dispersion of agent within the collagen-synthetic polymer conjugate. Alternatively, the agent may be covalently crosslinked to the collagen-synthetic polymer conjugate by means of a di- or multifunctionally activated synthetic polymer. Incorporation of biologically active molecules, and appropriate combinations of different biologically active molecules, can facilitate the anchoring, regrowth, and remodeling of a device or implant into normal tissue. Biologically active agents such as growth factors have been shown to retain their activity while bound to collagen-synthetic polymer conjugates. By tethering biologically active molecules to the implant, the amount of biologically active molecule required to achieve a desired effect is substantially reduced. Devices incorporating biologically active molecules such as cytokines or growth factors may serve as effective controlled release drug delivery systems. By varying the chemical linkage between the collagen and the synthetic polymer, it is possible to vary the effect with respect to release of the biologically active molecule. For example, when an "ester" linkage is used, the linkage is more easily broken under physiological conditions, allowing for sustained release of the biologically active molecule from the matrix. However, when an "ether" linkage is used, the bonds are not easily broken and the biologically active molecule will remain in place for longer periods of time with its active sites exposed, providing a biological effect on the natural substrate for the active site of the protein. It is possible to include a mixture of conjugates with different linkages so as to obtain variations in the effect with respect to the release of the biologically active molecule, *i.e.*, the sustained release or controlled release effect can be modified to obtain the desired rate of release. Methods of covalently binding biologically active agents to collagen-synthetic polymer conjugates are described in further detail in commonly owned U.S. Patent 5,162,430.

## EXAMPLES

The following examples are put forth so as to provide those of ordinary skill in the art with a complete disclosure and description of how to make the preferred embodiments of the conjugates, compositions, and devices and are not intended to limit the scope of what the inventors regard as their invention. Efforts have been made to ensure accuracy with respect to numbers used (*e.g.*, amounts, temperature, molecular weight, etc.) but some experimental errors and deviation should be accounted for. Unless indicated otherwise, parts are parts by weight, molecular weight is weight average molecular weight, temperature is in degrees Centigrade, and pressure is at or near atmospheric.

### Example 1

(Preparation of Improved Collagen-Synthetic Polymer Conjugates)

Ten (10) grams of atelopeptide fibrillar collagen, having a collagen concentration of 70 mg/ml, was acidifed to pH 4.5 by the addition of a total of 1.8 ml of 0.1 M HCl, in increments of 200 µl each, in an ice bath at 0 °C. The acidified collagen was subsequently adjusted to approximately pH 5 by adding a total of 7

ml of 0.02 M phosphate buffer (pH 10.5) without salt, then to approximately pH 6 by adding 0.4 ml of 0.2 M NaOH. The resulting collagen was placed in the refrigerator and allowed to incubate at 4°C overnight (approximately 16 hours). After overnight incubation, the pH of the collagen suspension was determined to be approximately 5.5.

A solution of difunctionally activated SG-PEG (MW ~3400) was prepared by adding 10 mg of SG-PEG to 0.1 ml of PBS. 0.9 ml of the collagen (pH 5.5) was mixed with 0.1 ml of the difunctionally activated SG-PEG solution using syringe-to-syringe mixing at room temperature. The PEG-collagen material was subsequently extruded into a rectangular mold having dimensions of 2 cm x 1 cm x 0.5 mm. Complete crosslinking of the PEG-collagen material in the mold appeared to have been achieved within approximately 4 to 5 hours. The crosslinked material was allowed to dry at room temperature overnight.

Example 2

(Preparation of Improved Collagen-Synthetic Polymer Conjugates)

Collagen adjusted to approximately pH 5.5 was prepared as described in the first paragraph of Example 1. A solution of difunctionally activated SG-PEG was prepared by adding 10 mg of SG-PEG to 0.1 ml of PBS. 0.9 ml of the collagen was mixed with 0.05 ml of 0.2 M NaOH, using syringe-to-syringe mixing at room temperature, to neutralize the pH to 7. The neutralized collagen was transferred into one syringe and immediately mixed with 0.1 ml of the difunctionally activated SG-PEG solution using syringe-to-syringe mixing. The PEG-collagen material was subsequently extruded into a rectangular mold having dimensions of 2 cm x 1 cm x 0.5 mm. Complete crosslinking of the PEG-collagen material in the mold appeared to have been achieved within approximately 30 minutes at room temperature. The crosslinked material was allowed to dry at room temperature overnight.

Example 3

(Determination of First Order Rate Constants for Hydrolysis of Difunctionally Activated SG-PEG)

Hydrolysis of the active group on SG-PEG (MW ~3400) was measured at pH 5.2, 7.5, and 8.5. Measurements were performed by HPLC under the conditions shown in Table 1.

Table 1

| HPLC Conditions for Determination of Hydrolysis of Difunctionally Activated SG-PEG | |
|---|---|
| Column | Waters® Ultrahydrogel 250 |
| Column Size | 7.8 mm x 30 cm |
| Pore Size | 250 Angstroms |
| Exclusion Limit | $8 \times 10^4$ |
| Injection Volume | 20 µl |
| Mobile Phase | 5 mM Sodium Acetate, pH 5.2 |
| Flow Rate | 0.5 ml/min |
| Pump Pressure | 1.5 mPa |
| Detection System | Dual Detector System of VV at 260 nm and Refractive Index |
| Sample Concentration | 5.0 mg/ml |

Fifteen (15.0) milligrams of difunctionally activated SG-PEG (MW 3400) was added to 3 ml of 0.2 M phosphate buffer at the appropriate pH (5.2, 7.5, or 8.5). The resulting solution was mixed. Twenty (20) microliters of each solution was injected into the column at room temperature. The peak retention time for difunctionally activated SG-PEG was determined to be 17 minutes. First order rate constants for hydrolysis ($K_h$) of difunctionally activated SG-PEG at pH 5.2, 7.5, and 8.5 were calculated and are presented in Table 2.

Table 2

| First Order Rate Constants for Hydrolysis of Difunctionally Activated SG-PEG | | | |
|---|---|---|---|
| pH | Temperature (°C) | $K_h$ ($min^{-1}$) x $10^3$ | $t_{1/2}$(min) |
| 5.2 | 22 | 5.7 | 121 |
| 7.5 | 22 | 18.5 | 37 |
| 8.5 | 22 | 73.5 | 9.4 |

Example 4

(DSC Measurements of Collagens Having Controlled Fiber Size Distributions)

Fibrillar collagen (35 mg/ml collagen concentration) at pH 7.2 was used to prepare reprecipitated collagen samples at pH 3.0, 4.0, 5.0, 5.5, and 6.3, respectively. The pH 3.0 collagen sample was prepared by mixing 10 ml of fibrillar collagen with 0.6 ml of 1 M HCl. The pH 6.3 sample was prepared by mixing 10 ml of fibrillar collagen with 0.15 ml of 1 M HCl. The pH 4.0 sample was prepared by mixing 1 ml of the pH 3 collagen sample with 1 ml of the pH 6.3 sample. The pH 5.0 sample was prepared by mixing 1 ml of the pH 3.0 sample with 2 ml of the pH 6.3 sample. The pH 5.5 sample was prepared by mixing 1 ml of the pH 5.0 sample with 0.4 ml of the pH 6.3 sample. An additional pH 7.2 sample was prepared to contain 0.26 M NaCl in order to determine the effect of salt on the collagen.

The seven collagen samples were compared using differential scanning calorimetry (DSC). DSC measurements are presented in Table 3 and Figures 1 - 7 for the collagen samples at pH 3.0, 4.0, 5.0, 5.5, 6.3, 7.2, and 7.2 with 0.26 M NaCl, respectively.

Table 3

| DSC Measurements of Fibrillar and Reprecipitated Collagens | |
|---|---|
| pH of Collagen Sample | DSC Measurement (°C) |
| 3.0 | 40.0 |
| 4.0 | 42.9 |
| 5.0 | 51.9 |
| 5.5 | 55.9 |
| 6.3 | 56.1 |
| 7.2 | 56.9 |
| 7.2 with 0.26 M NaCl | 45.1, 54.5 |

Differential scanning calorimetry is used to measure denaturational transitions in fibrillar collagen. As shown in Table 3, above, and Figures 1 - 7, there was a slight increase in tthe melting temperature of the collagen from pH 5.5, to 6.3, to 7.2. As the pH increases, a shoulder is added at higher temperature, indicating that fibrillogenesis is proceeding. On the other hand, the DSC measurements for the collagen samples at pH 3.0, 4.0, and 5.0 cover a broad range of temperatures, indicating the progression of fibrillogenesis, from nonfibrillar collagen to small fibrils, as the pH of the collagen composition is increased. It must be noted that the DSC measurement of the pH 7.2 collagen sample containing 0.26 M NaCl is shown in Figure 7 to have multiple denaturation temperatures with two major endotherms, indicating the disruption of the collagen fiber structure and partial disassembly of the collagen fibers by salt.

Example 5

(Fiber Size Distributions of Reprecipitated Collagen Compositions)

Reprecipitated collagen samples at pH 3.0, 4.0, 5.0, 5.5, and 6.3, respectively, were prepared as described in Example 4, above, using fibrillar collagen (35 mg/ml collagen concentration) at pH 7.3 as the starting material. In order to provide samples dilute enough for fiber size analysis, while maintaining the

desired pH of the sample, the collagen samples were diluted to a concentration of 0.003% (weight per volume) solids in various buffers, as follows: The pH 3.0 collagen sample was diluted in a 0.1 M HCl buffer. The pH 4.0 sample was diluted in a 0.001 M NaAc / HAc buffer. The pH 5.0 sample was diluted in a 0.02 M NaAc / HAc buffer. The pH 5.5, 6.3, and 7.3 samples were all diluted in a 0.02 M $Na_2HPO_4$ / $NaH_2PO_4$ buffer. An additional pH 7.3 sample was diluted in the 0.02 M $Na_2HPO_4$ / $NaH_2PO_4$ buffer, with the addition of 0.26 M NaCl to determine the effect of salt on the fiber size distribution of collagen.

The fiber size distributions of the seven collagen samples were measured by light blockage using the AccuSizer® 770 Optical Particle Sizer, Software C770, Version 2.0 (manufactured by Particle Sizing Systems, Inc., Santa Barbara, CA). Fiber size population distributions are shown in Figures 8 - 14, respectively, for the collagen samples at pH 3.0, 4.0, 5.0, 5.5, 6, 7.2, and 7.3 with 0.26 M NaCl. Table 5 lists the number-weighted mean fiber size in microns for each of the samples. Table 5 also lists the fiber size, in microns, under which 80% of the fibers fall for each of the samples.

Table 5

| Fiber Size Distributions of Various Collagen Compositions | | |
|---|---|---|
| pH of Collagen Sample | Mean Fiber Size of Sample (µm) | 80% of Fibers in Sample < (µm) |
| 3.0 | 6.34 | 8.05 |
| 4.0 | 6.56 | 8.12 |
| 5.0 | 7.04 | 8.32 |
| 5.5 | 6.63 | 8.24 |
| 6.3 | 12.96 | 16.03 |
| 7.3 | 12.17 | 15.56 |
| 7.3 with 0.26 M NaCl | 10.24 | 10.77 |

As shown by the fiber size distribution results presented in Table 5, above, the collagen samples at pH 3.0, 4.0, 5.0, and 5.5 show a gradual, though small, increase in mean fiber size with increasing pH. By contrast, the pH 6.3 and 7.3 collagen samples show a greatly increased mean fiber size, almost double that of the lower pH samples, indicating that fibrillogenesis is basically complete by about pH 6.

The pH 7.3 collagen sample with 0.26 M NaCl showed a slight decrease in mean fiber size as compared to the pH 7.3 sample with no salt, indicating that the salt has prevented assembly of the collagen molecules into thick fibers. Increasing the amount of salt added to the composition would likely result in an even smaller mean collagen fiber size.

As shown in Figures 8 - 11, the pH 3.0, 4.0, 5.0, and 5.5 collagen samples have relatively narrow fiber size population distributions. By comparison, the fiber size population distributions shown in Figures 12 and 13, respectively, for the pH 6.3 and 7.3 collagen samples are quite broad, with a long tail on the right-hand (far) end of the curve, indicating the presence of very large fibers. Although the mean fiber size is somewhat smaller than those of the pH 6.3 and 7.3 samples, the fiber size population distribution for the pH 7.3 collagen sample with 0.26 M NaCl, shown in Figure 14, also has a long tail on the far end of the curve, indicating incomplete fiber disassembly of the collagen by the salt. Again, addition of more salt could potentially eliminate these larger fibers which fall into the tail end of the fiber size population distribution curve.

## Claims

1. A conjugate comprising collagen covalently bound to a synthetic hydrophilic polymer, wherein the collagen comprises fibers, at least 80% of which collagen fibers have a fiber size of about 10 µm or less.

2. An optically clear conjugate comprising collagen covalently bound to a synthetic hydrophilic polymer, wherein the collagen comprises fibers, at least 80% of which collagen fibers have a fiber size of about 8.0 µm or less.

3. A conjugate comprising collagen covalently bound to a synthetic hydrophilic polymer, wherein the collagen comprises fibers having a mean fiber size between about 5.0 and about 8.0 µm.

4. The conjugate of any one of the preceding claims, wherein the collagen is atelopeptide collagen.

5. The conjugate of any one of the preceding claims, wherein the synthetic hydrophilic polymer is a functionally activated, preferably a difunctionally activated, polyethylene glycol.

6. The conjugate of any one of the preceding claims, wherein the collagen and the synthetic hydrophilic polymer are covalently bound by means of a linkage selected from the group consisting of an ether linkage, an ester linkage, and a urethane linkage.

7. The conjugate of any one of the preceding claims, wherein 80% of the collagen fibers have a fiber size of about 8.5 µm or less, and preferably of about 8.0 µm or less.

8. The conjugate of any one of the preceding claims, wherein the conjugate is translucent.

9. The conjugate of any one of claims 1 to 7, wherein the conjugate is optically clear.

10. An implant composition comprising a conjugate comprising collagen covalently bound to a synthetic hydrophilic polymer, wherein the collagen comprises fibers, at least 80% of which collagen fibers have a fiber size of about 10 µm or less.

11. The implant of claim 10, wherein the collagen is atelopeptide collagen.

12. The implant of claim 10 or 11, wherein the synthetic hydrophilic polymer is a functionally activated, preferably a difunctionally activated, polyethylene glycol.

13. The implant of any one of claims 10 to 12, wherein the collagen and the synthetic hydrophilic polymer are covalently bound by means of a linkage selected from the group consisting of an ether linkage, an ester linkage, and a urethane linkage.

14. The implant of claim 20, wherein 80% of the collagen fibers have a fiber size of about 8.5 µm or less, and preferable of about 8.0 µm or less.

15. The implant of any one of claims 10 to 14, wherein the conjugate is translucent.

16. The imlant of any one of claims 10 to 14, wherein the conjugate is optically clear.

17. A process for preparing an improved collagen-synthetic polymer conjugate comprising the steps of:
providing a suspension comprising collagen fibers, at least 80% of which collagen fibers have a fiber size of about 10 µm or less;
providing a solution of a synthetic hydrophilic polymer;
mixing the collagen with the synthetic hydrophilic polymer solution; and allowing the synthetic hydrophilic polymer to covalently bind with the collagen.

18. The process of claim 17, wherein the collagen suspension is prepared by reprecipitation of fibrillar collagen.

19. The process of claim 17, wherein the collagen suspension is prepared by addition of salt to fibrillar collagen.

20. A process for preparing an improved collagen-synthetic polymer conjugate comprising the steps of:
providing a suspension of reprecipitated collagen having a pH of about 6 or less;
providing a solution of a synthetic hydrophilic polymer;
mixing the collagen with the synthetic hydrophilic polymer solution; and
allowing the synthetic hydrophilic polymer to covalently bind with the collagen.

21. The process of claim 20, additionally comprising the step of adjusting the pH of the reprecipitated collagen to within the range of between about 6 and about 9 prior to mixing the collagen with the synthetic hydrophilic polymer.

**22.** A process for preparing an improved collagen-synthetic polymer conjugate comprising the steps of:
providing a suspension of fibrillar collagen having a pH within the range of between about 6 and about 9;
adjusting the pH of the collagen suspension to about 5 or less;
allowing the collagen suspension to incubate for a sufficient time to effect fiber disassembly of the collagen;
adjusting the pH of the collagen suspension to between about 5 and about 6 to produce reprecipitated collagen;
allowing the reprecipitated collagen to incubate for a sufficient time to achieve the desired fiber size;
providing a solution of a synthetic hydrophilic polymer;
adjusting the pH of the reprecipitated collagen to within the range of between about 6 and about 9;
immediately mixing the collagen with the synthetic hydrophilic polymer solution; and
allowing the synthetic hydrophilic polymer to covalently bind with the collagen.

**23.** A process for preparing an improved collagen-synthetic polymer conjugate comprising the steps of:
providing a suspension of fibrillar collagen having a pH within the range of between about 6 and about 9;
adjusting the pH of the collagen suspension to about 5 or less;
allowing the collagen suspension to incubate for a sufficient time to effect fiber disassembly of the collagen;
adjusting the pH of the collagen suspension to between about 5 and about 6 to produce reprecipitated collagen;
allowing the reprecipitated collagen to incubate for a sufficient time to achieve the desired fiber size;
providing a solution of a synthetic hydrophilic polymer;
mixing the collagen with the synthetic hydrophilic polymer solution; and
allowing the synthetic hydrophilic polymer to covalently bind with the collagen.

**24.** A process for preparing an optically clear nonfibrillar collagen-synthetic polymer conjugate comprising the steps of:
providing a suspension of fibrillar collagen having a pH within the range of between about 6 and about 9;
adjusting the pH of the collagen suspension to about 5 or less;
allowing the collagen suspension to incubate for a sufficient time at a temperature within the range of between about 0 °C and about 8 °C to produce nonfibrillar collagen;
providing a solution of a synthetic hydrophilic polymer;
mixing the nonfibrillar collagen with the synthetic hydrophilic polymer solution at a temperature within the range of between about 0 °C and about 8 °C; and
allowing the synthetic hydrophilic polymer to covalently bind with the nonfibrillar collagen.

**25.** A process for preparing an improved collagen-synthetic polymer conjugate comprising the steps of:
providing a suspension of fibrillar collagen having a pH within the range of between about 6 and about 9;
adding salt to the collagen suspension in an amount sufficient to reduce the fiber size of the collagen such that at least 80% of the collagen fibers in the collagen suspension have a fiber size of about 10 μm or less;
providing a solution of a synthetic hydrophilic polymer;
mixing the collagen with the synthetic hydrophilic polymer solution; and
allowing the synthetic hydrophilic polymer to covalently bind with the collagen.

**26.** The process of claim 19 or 25, wherein the salt is sodium chloride.

**27.** The process of any one of claims 22 to 26, wherein the suspension of fibrillar collagen has a pH of approximately 7.

**28.** The process of any one of claims 22 to 24, wherein the pH of the collagen is adjusted to approximately 7 prior to mixing with the synthetic hydrophilic polymer solution.

**29.** The process of any one of claims 20 to 28, wherein the solution of synthetic hydrophilic polymer has a pH within the range of about 8 to about 9.5.

**30.** The process of any one of claims 22 to 24, wherein the pH of the collagen suspension is adjusted to between about 3 and about 5 to effect fiber disassembly.

**31.** The process of claim 24, additionally comprising the step of adjusting the pH of the nonfibrillar collagen to within the range of between about 6 and about 9 prior to mixing the collagen with the synthetic hydrophilic polymer.

**32.** The process of any one of claims 17 to 31, wherein the collagen is an atelopeptide fibrillar collagen.

**33.** The process of any one of claims 17 to 32, wherein the collagen suspension has a collagen concentration within the range of between about 10 mg/ml and about 120 mg/ml.

**34.** The process of any one of claims 17 to 33, wherein the synthetic hydrophilic polymer is a functionally activated, preferably a difunctionally activated, polyethylene glycol.

**35.** The process of any one of claims 17 to 34, wherein the collagen and the synthetic hydrophilic polymer are covalently bound by means of a linkage selected from the group consisting of an ether linkage, an ester linkage, and a urethane linkage.

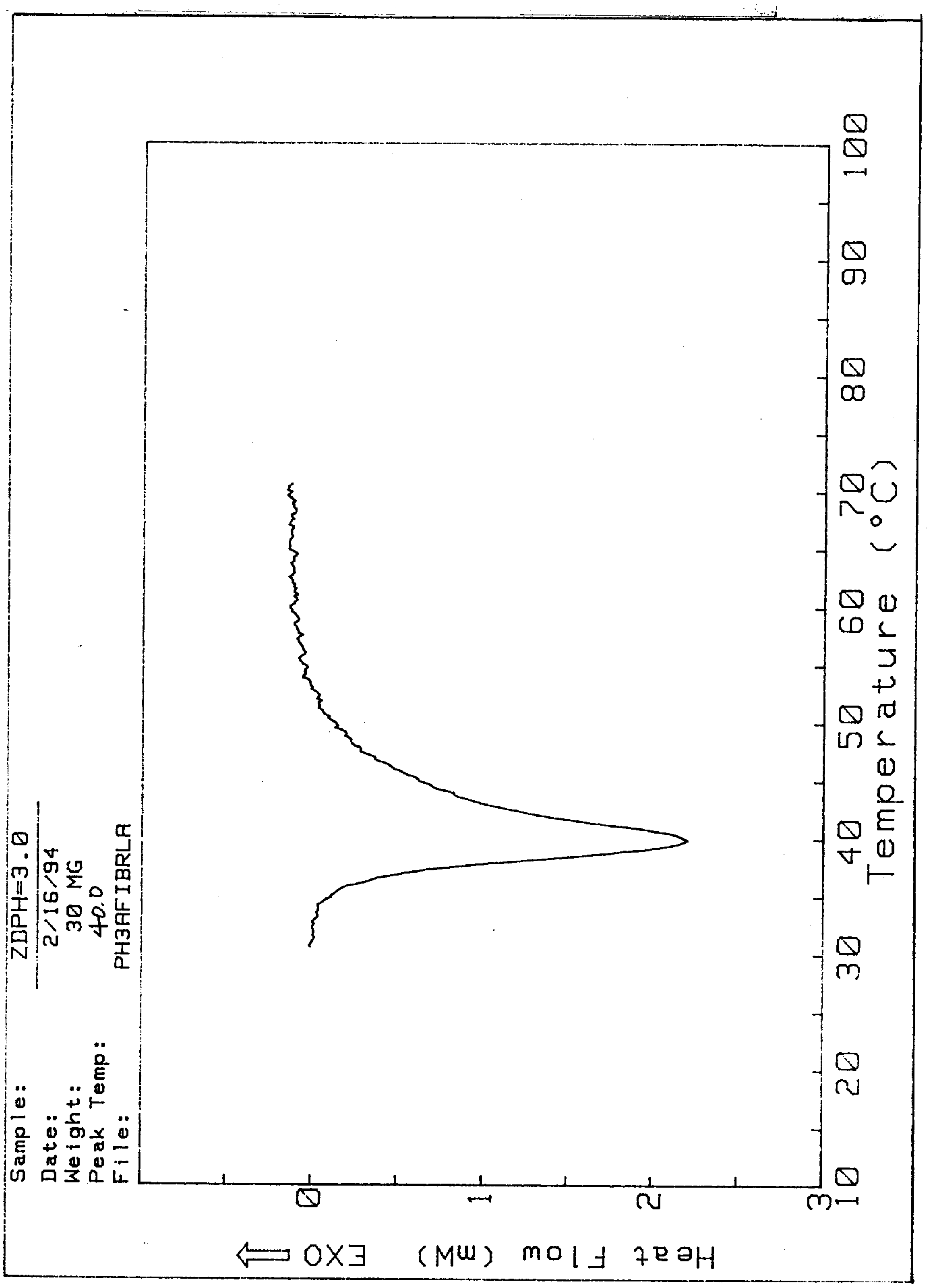
Sample: ZDPH=3.0
Date: 2/16/94
Weight: 30 MG
Peak Temp: 40.0
File: PH3AFIBRLA
Heat Flow (mW) EXO
0
1
2
3
10
20
30
40
50
60
70
80
90
100
Temperature (°C)

Figure 1.

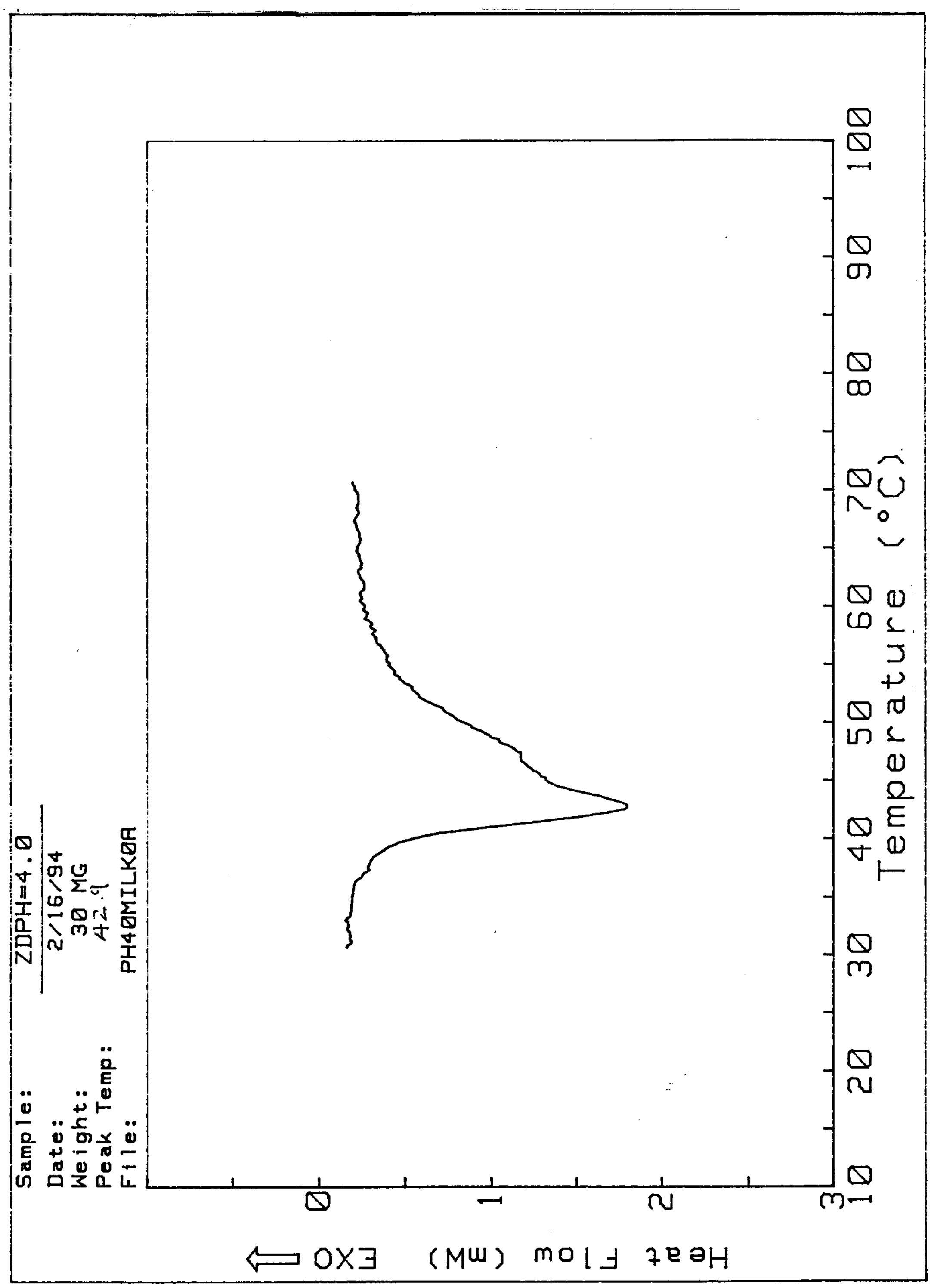
Sample: ZDPH=4.0
Date: 2/16/94
Weight: 30 MG
Peak Temp: 42.9
File: PH40MILK0A
Heat Flow (mW) EXO
0
1
2
3
10
20
30
40
50
60
70
80
90
100
Temperature (°C)

Figure 2

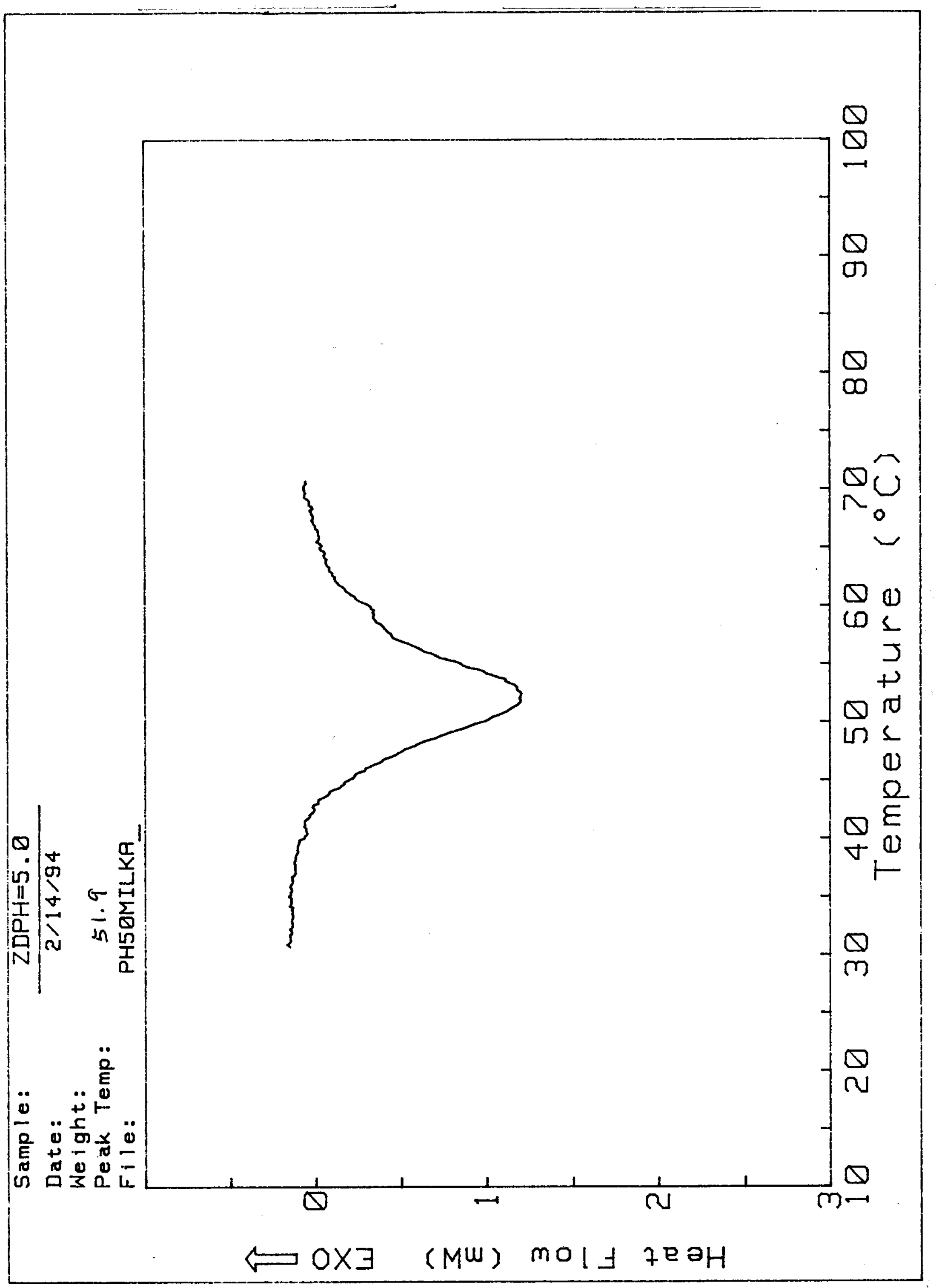

Sample: ZDPH=5.0
Date: 2/14/94
Weight:
Peak Temp: 51.9
File: PH50MILKA
Heat Flow (mW) EXO
0
1
2
3
10
20
30
40
50
60
70
80
90
100
Temperature (°C)


Figure 3.

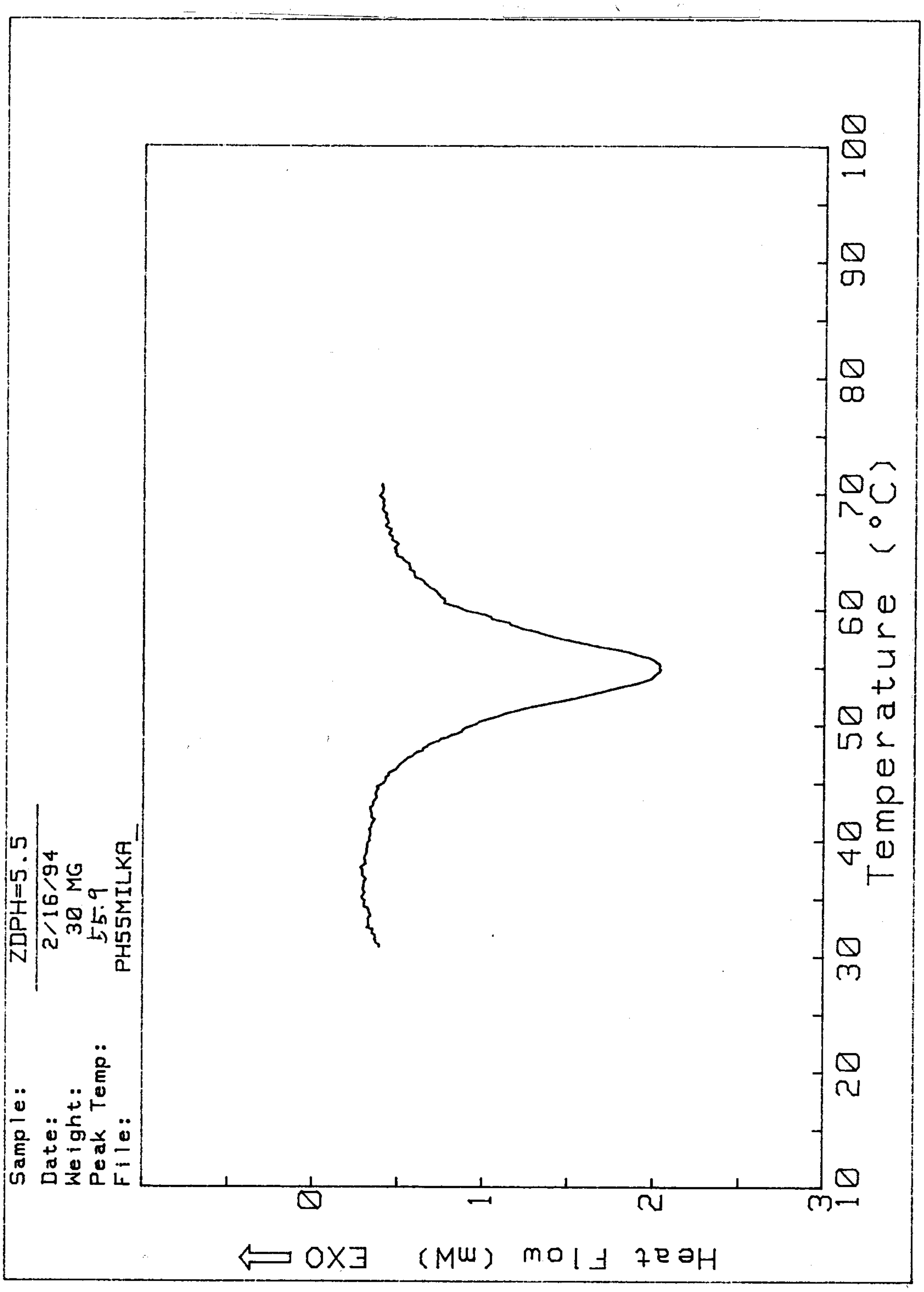
Sample: ZDPH=5.5
Date: 2/16/94
Weight: 30 MG
Peak Temp: 55.9
File: PH55MILKA
Heat Flow (mW) EXO
Temperature (°C)
0
1
2
3
10
20
30
40
50
60
70
80
90
100

Figure 4.

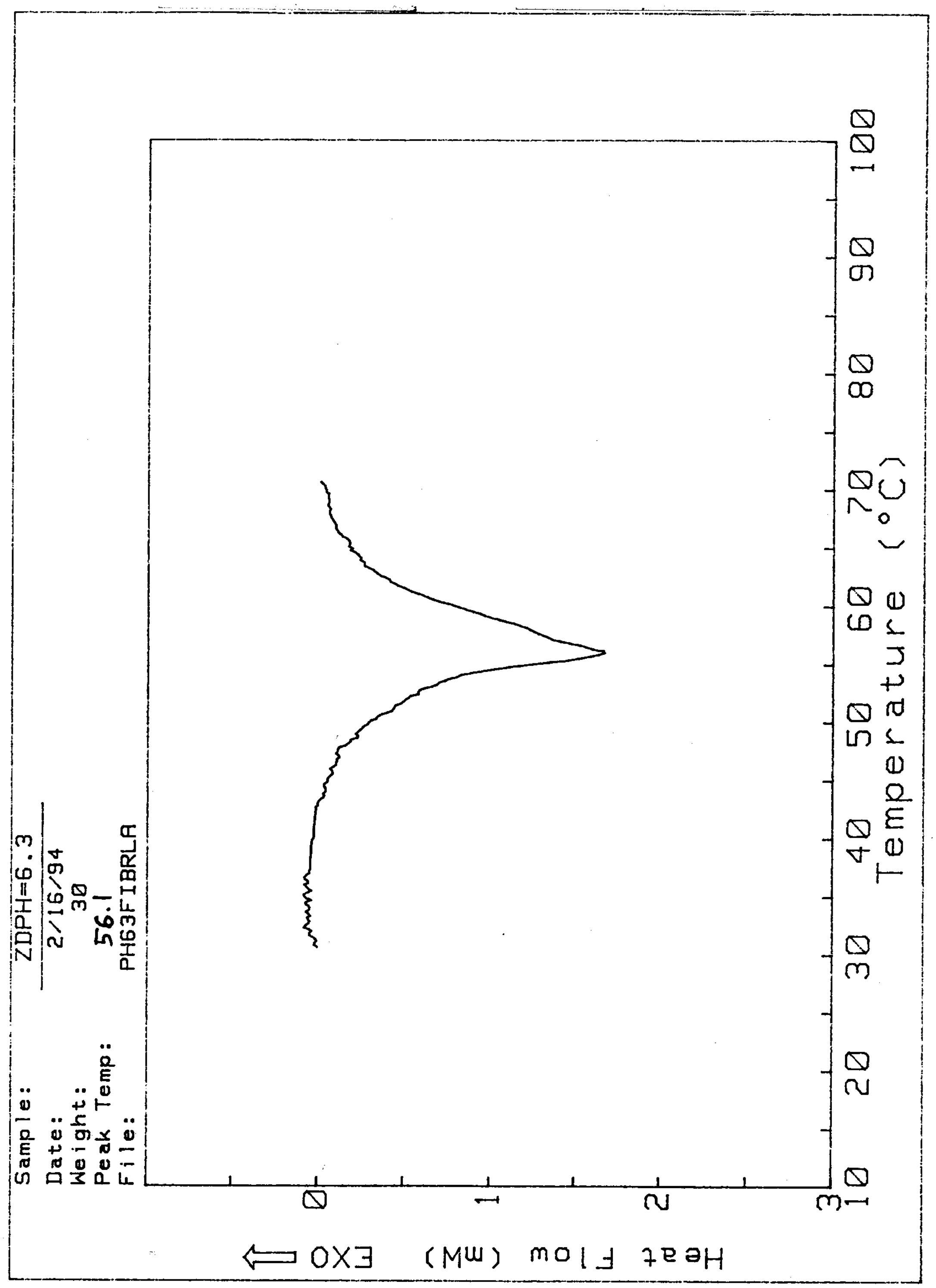
Sample: ZDPH=6.3
Date: 2/16/94
Weight: 30
Peak Temp: 56.1
File: PH63FIBRLA
Heat Flow (mW) EXO
0
1
2
3
10
20
30
40
50
60
70
80
90
100
Temperature (°C)

Figure 5.

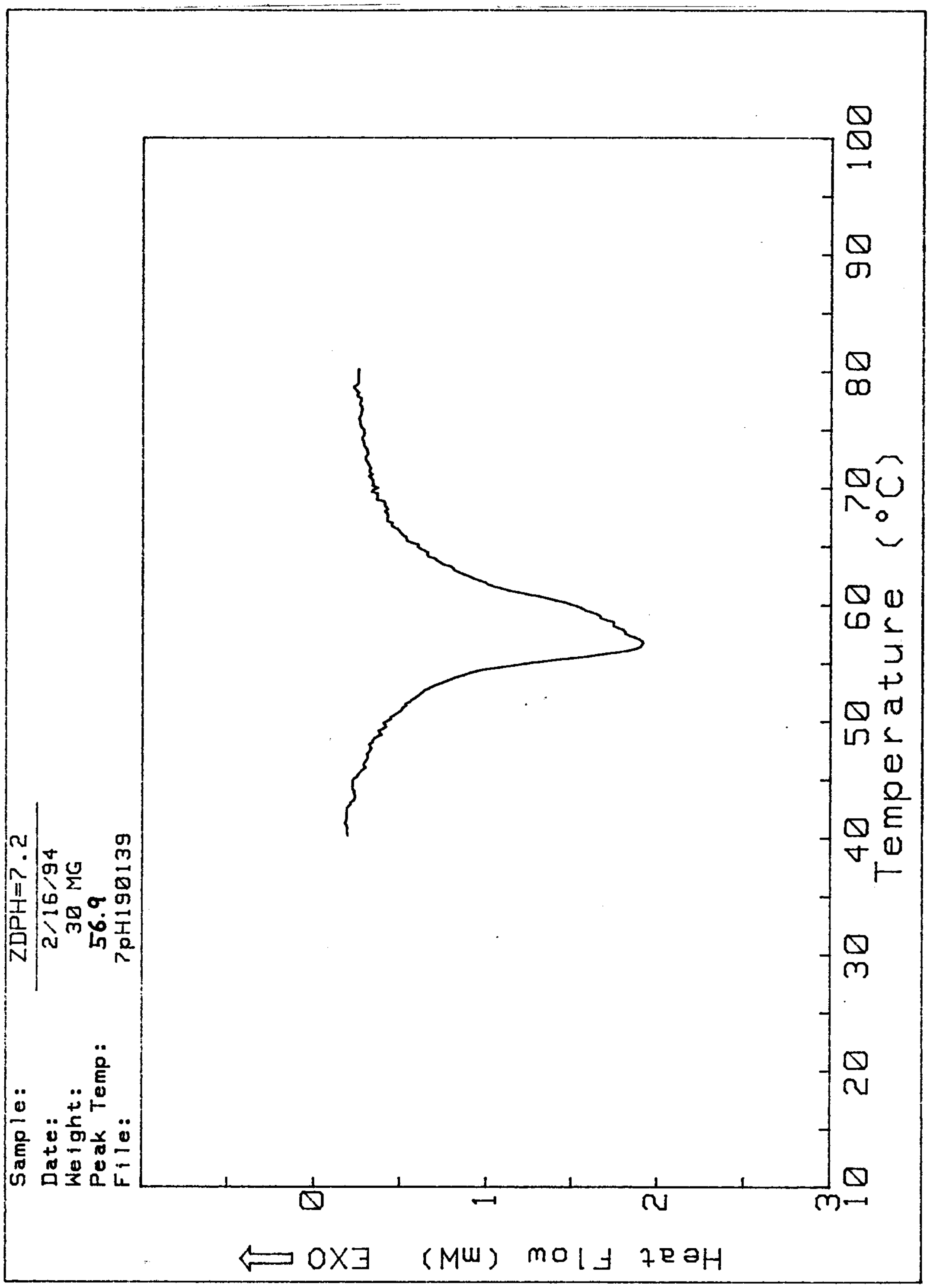
Sample: ZDPH=7.2
Date: 2/16/94
Weight: 30 MG
Peak Temp: 56.9
File: 7pH190139
Heat Flow (mW) EXO
0
1
2
3
10
20
30
40
50
60
70
80
90
100
Temperature (°C)

Figure 6

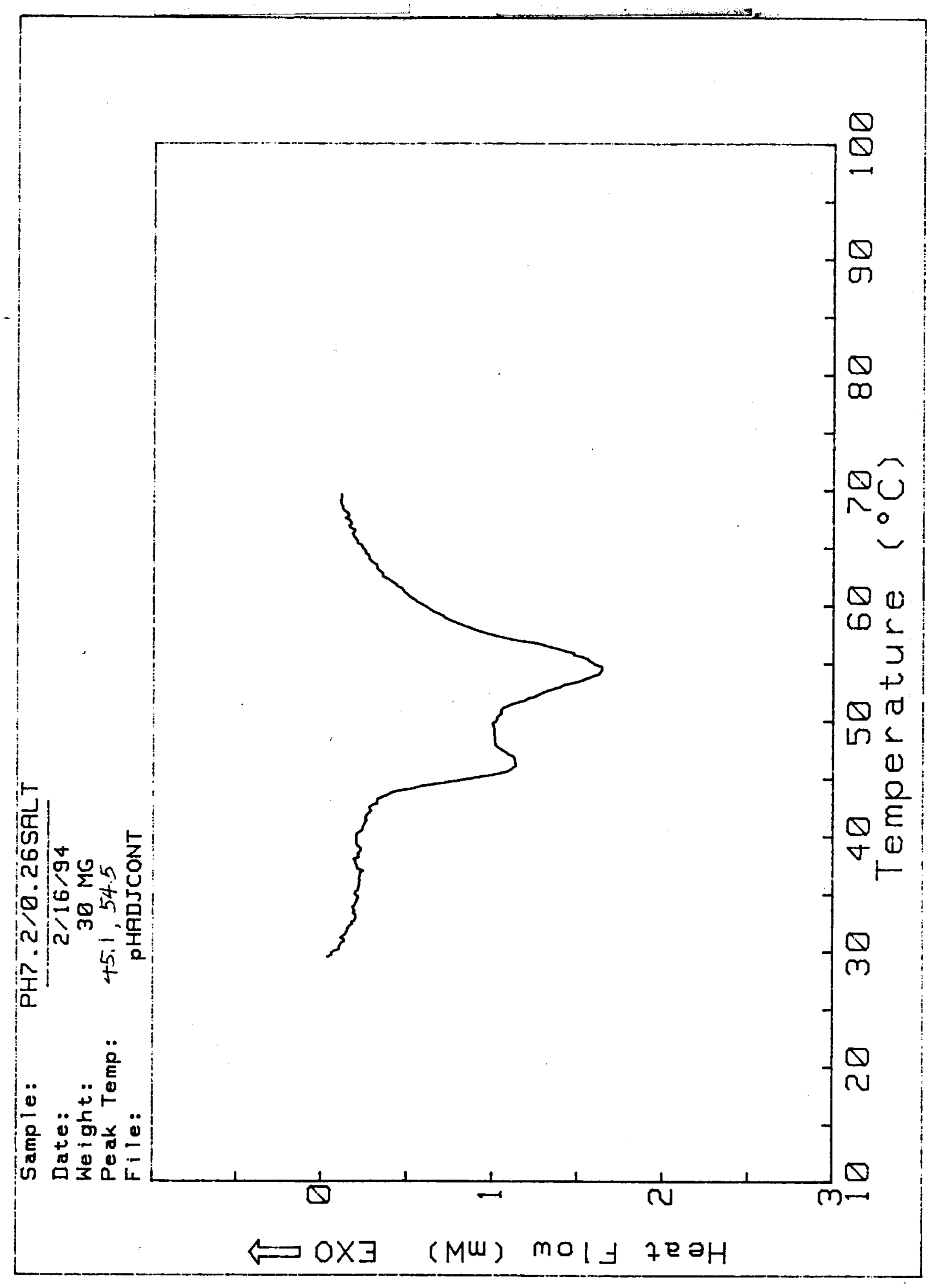
Sample: PH7.2/0.26SALT
Date: 2/16/94
Weight: 30 MG
Peak Temp: 45.1, 54.5
File: pHADJCONT
Heat Flow (mW) EXO
0
1
2
3
10 20 30 40 50 60 70 80 90 100
Temperature (°C)

Figure 7

PARTICLE SIZING SYSTEMS
Santa Barbara, California

Model 770 AccuSizer

COLLAGEN SAMPLE 1 PH=3
File Name = RHEE.1 Time Date = 15:51 01/31/1994
Sensor Model: hr4001d S/N: 9212.007 Cal. File: 770DEMO.SNS

Elapsed Time of Data Collection (min:sec)= 1:0
Actual No. of Particles Sized = 133184
Approximate Total No. of Particles in Sample = 206469
Dilution Factor (approx.) = 1.550
Total Fluid Volume Sampled = 60.0 ml
Number of Diameter Channels = 128
Num. Wt. Mean = 6.34 um Vol. Wt. Mean = 56.35 um (0.000 % Threshold)

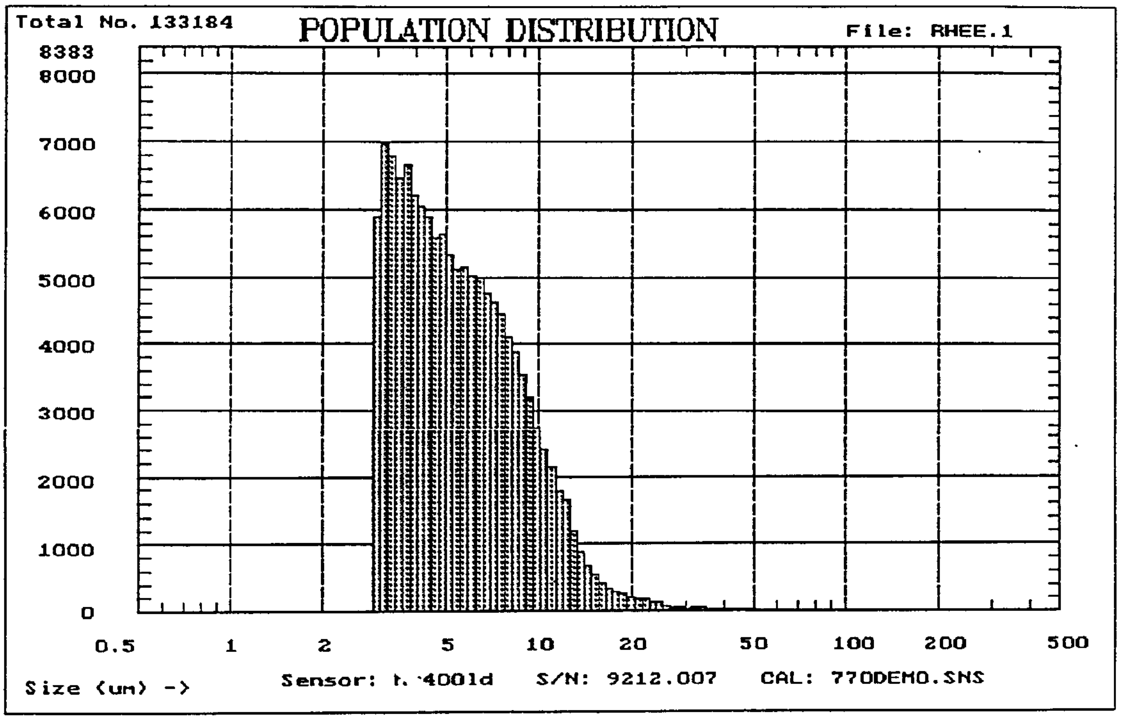


File Name: RHEE.1

Figure 8

PARTICLE SIZING SYSTEMS
Santa Barbara, California

Model 770 AccuSizer

COLLAGEN SAMPLE 2 PH=4
File Name = RHEE.21 Time Date = 16:05 01/31/1994
Sensor Model: hr400ld S/N: 9212.007 Cal. File: 770DEMO.SNS

Elapsed Time of Data Collection (min:sec)= 1:0
Actual No. of Particles Sized = 358557
Approximate Total No. of Particles in Sample = 578523
Dilution Factor (approx.) = 1.613
Total Fluid Volume Sampled = 60.0 ml
Number of Diameter Channels = 128
Num. Wt. Mean = 6.56 um Vol. Wt. Mean = 52.78 um (0.000 % Threshold)

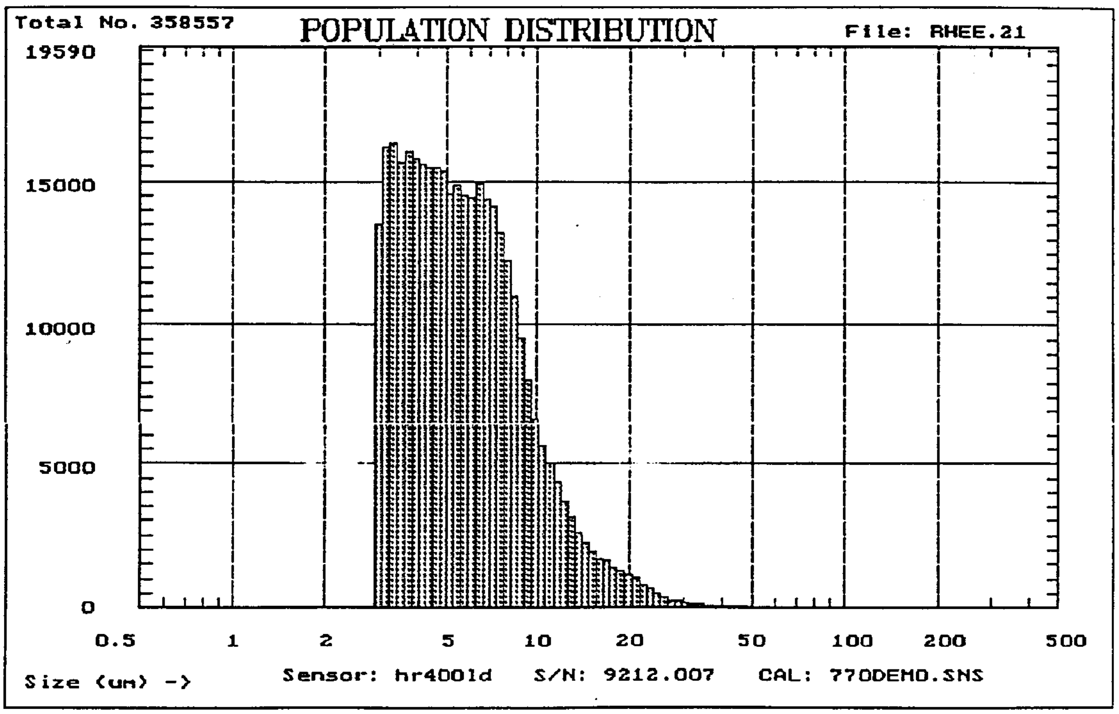


File Name: RHEE.21

Figure 9.

PARTICLE SIZING SYSTEMS
Santa Barbara, California

Model 770 AccuSizer

COLLAGEN SAMPLE 3 PH=5
File Name = RHEE.31 Time Date = 16:20 01/31/1994
Sensor Model: hr4001d S/N: 9212.007 Cal. File: 770DEMO.SNS

Elapsed Time of Data Collection (min:sec)= 1:0
Actual No. of Particles Sized = 370318
Approximate Total No. of Particles in Sample = 562701
Dilution Factor (approx.) = 1.520
Total Fluid Volume Sampled = 60.0 ml
Number of Diameter Channels = 128
Num. Wt. Mean = 7.04 um Vol. Wt. Mean = 99.84 um (0.000 % Threshold)

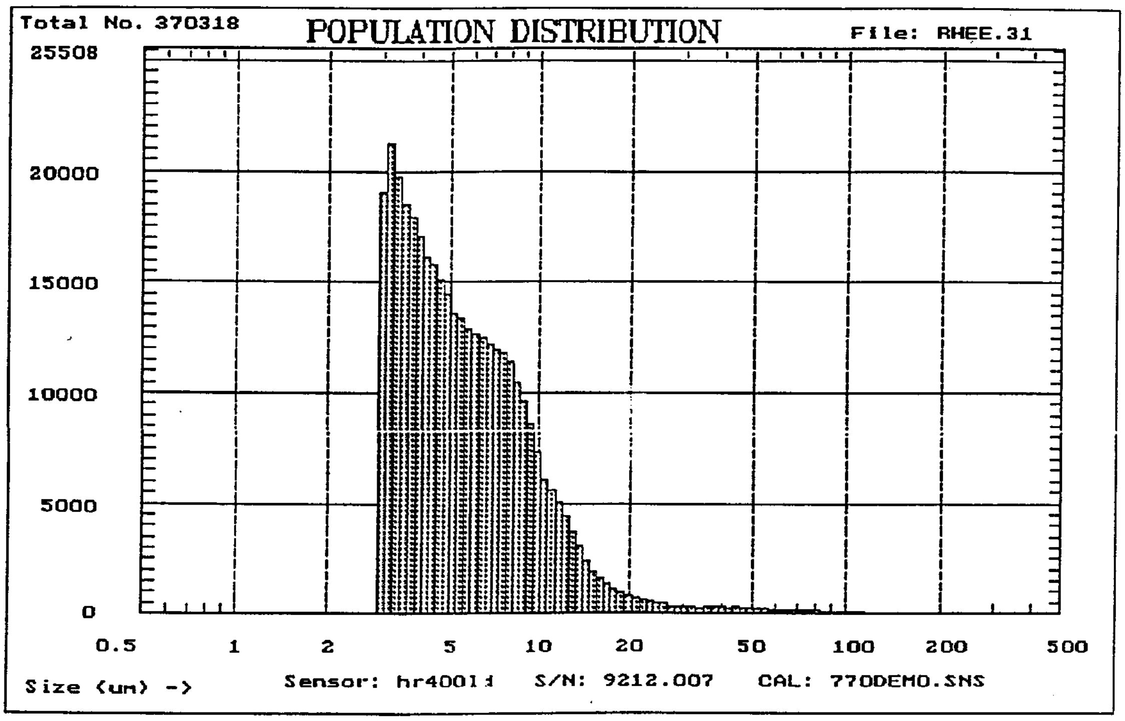


File Name: RHEE.31

Figure 1C.

PARTICLE SIZING SYSTEMS
Santa Barbara, California

Model 770 AccuSizer

COLLAGEN SAMPLE 8 PH=5.5
File Name = RHEE.83 Time Date = 17:43 02/02/1994
Sensor Model: hr4001d S/N: 9212.007 Cal. File: 770DEMO.SNS

Elapsed Time of Data Collection (min:sec)= 1:0
Actual No. of Particles Sized = 400729
Approximate Total No. of Particles in Sample = 1131915
Dilution Factor (approx.) = 2.825
Total Fluid Volume Sampled = 60.0 ml
Number of Diameter Channels = 128
Num. Wt. Mean = 6.63 um Vol. Wt. Mean = 120.04 um (0.000 % Threshold)

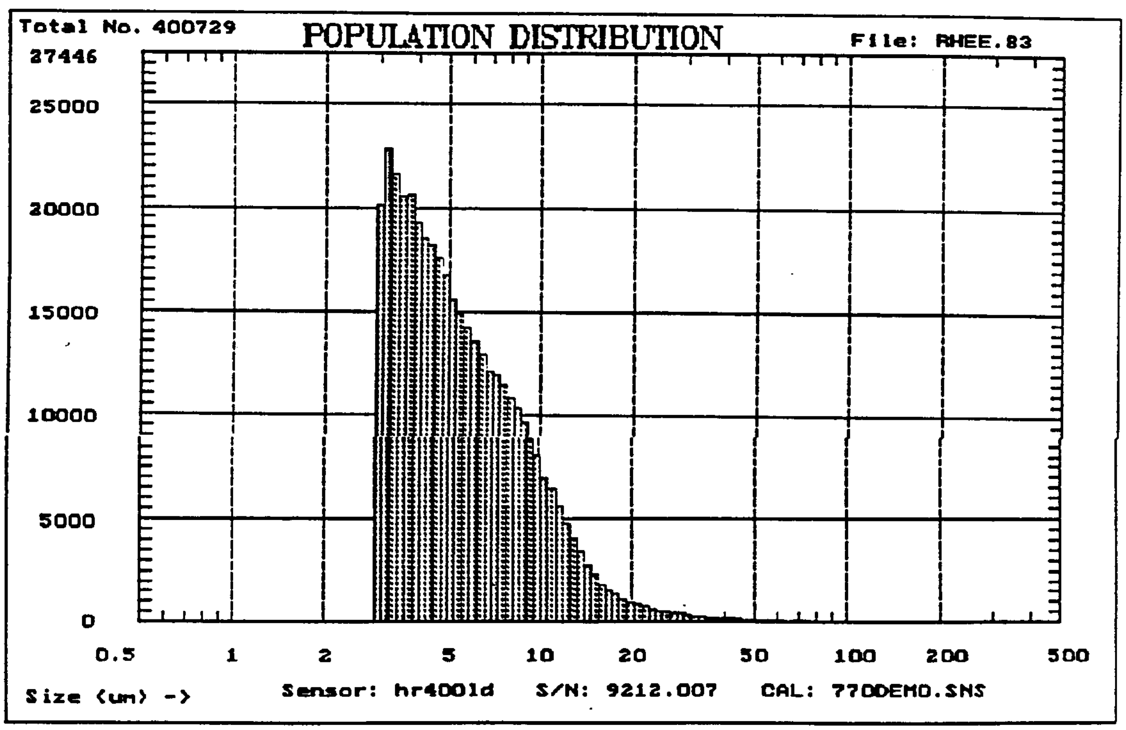


File Name: RHEE.83

Figure 11.

PARTICLE SIZING SYSTEMS
Santa Barbara, California

Model 770 AccuSizer

COLLAGEN SAMPLE 5 PH=6.3
File Name = RHEE.51 Time Date = 17:40 01/31/1994
Sensor Model: hr4001d S/N: 9212.007 Cal. File: 770DEMO.SNS

Elapsed Time of Data Collection (min:sec)= 1:0
Actual No. of Particles Sized = 237138
Approximate Total No. of Particles in Sample = 353196
Dilution Factor (approx.) = 1.489
Total Fluid Volume Sampled = 60.0 ml
Number of Diameter Channels = 128
Num. Wt. Mean = 12.96 um Vol. Wt. Mean = 145.05 um (0.000 % Threshold)

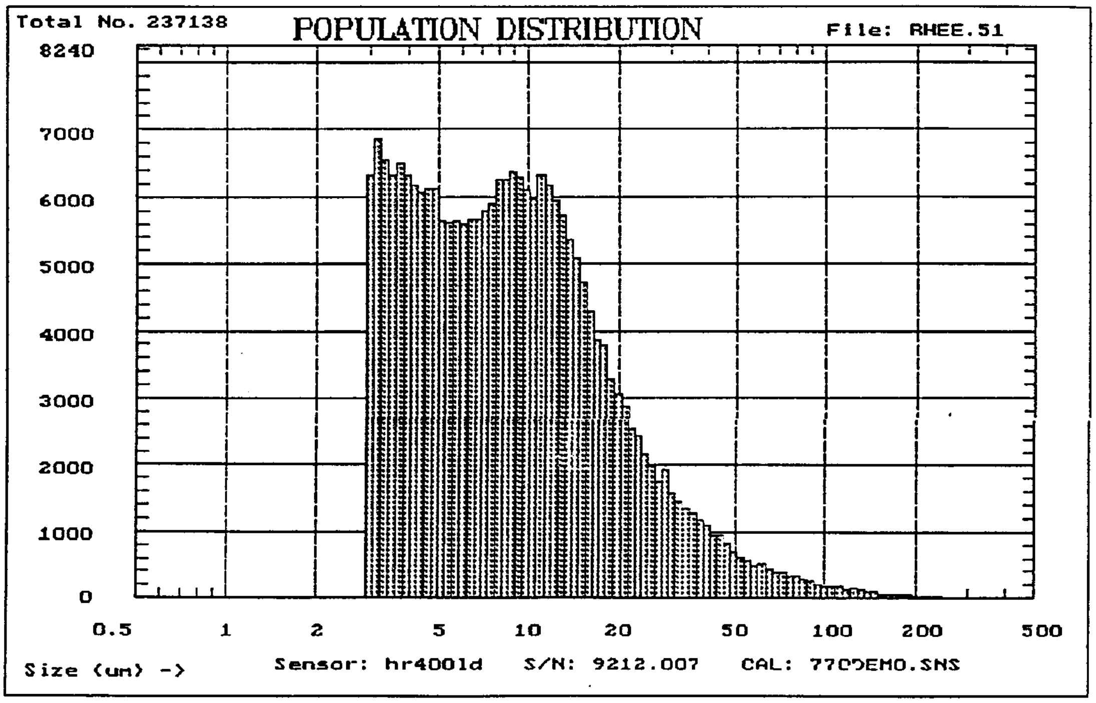


File Name: RHEE.51

Figure 12.

PARTICLE SIZING SYSTEMS
Santa Barbara, California

Model 770 AccuSizer

COLLAGEN SAMPLE 6 PH=7.3
File Name = RHEE.61 Time Date = 18:33 01/31/1994
Sensor Model: hr400ld S/N: 9212.007 Cal. File: 770DEMO.SNS

Elapsed Time of Data Collection (min:sec)= 1:0
Actual No. of Particles Sized = 412696
Approximate Total No. of Particles in Sample = 2810430
Dilution Factor (approx.) = 6.810
Total Fluid Volume Sampled = 60.0 ml
Number of Diameter Channels = 128
Num. Wt. Mean = 12.17 um Vol. Wt. Mean = 118.90 um (0.000 % Threshold)

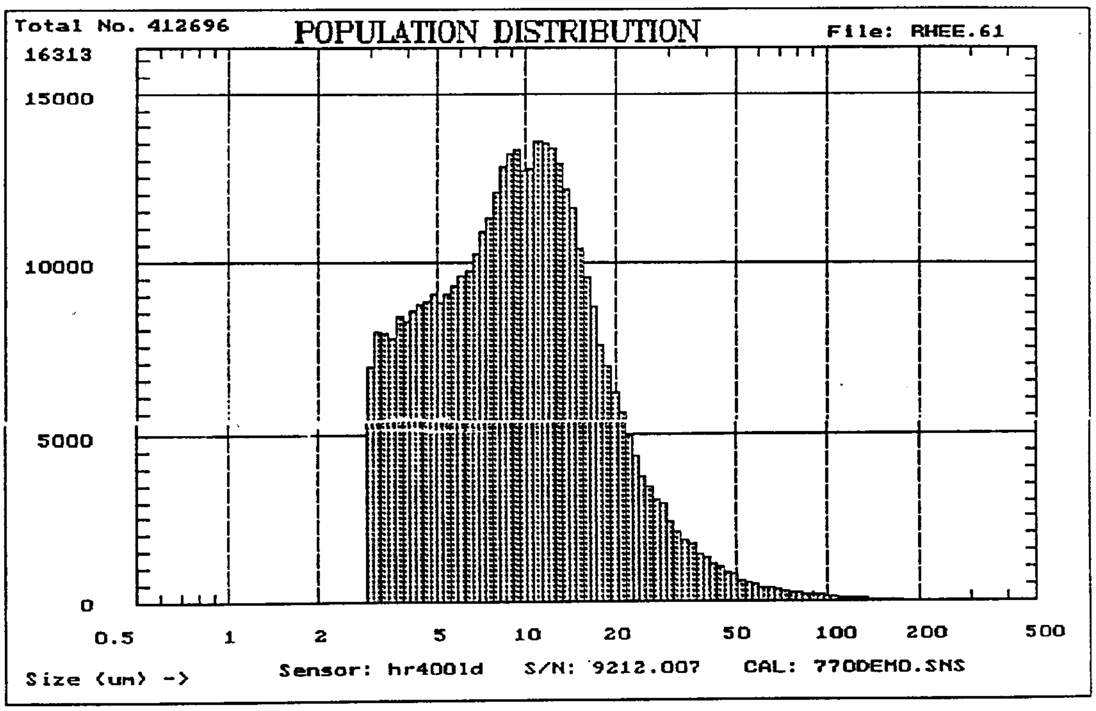


File Name: RHEE.61

Figure 13.

PARTICLE SIZING SYSTEMS
Santa Barbara, California

Model 770 AccuSizer

COLLAGEN SAMPLE 7 PH=7.3
File Name = RHEE.71 Time Date = 18:02 01/31/1994
Sensor Model: hr400ld S/N: 9212.007 Cal. File: 770DEMO.SNS

Elapsed Time of Data Collection (min:sec)= 1:0
Actual No. of Particles Sized = 377547
Approximate Total No. of Particles in Sample = 585269
Dilution Factor (approx.) = 1.550
Total Fluid Volume Sampled = 60.0 ml
Number of Diameter Channels = 128
Num. Wt. Mean = 10.24 um Vol. Wt. Mean = 96.25 um (0.000 % Threshold)

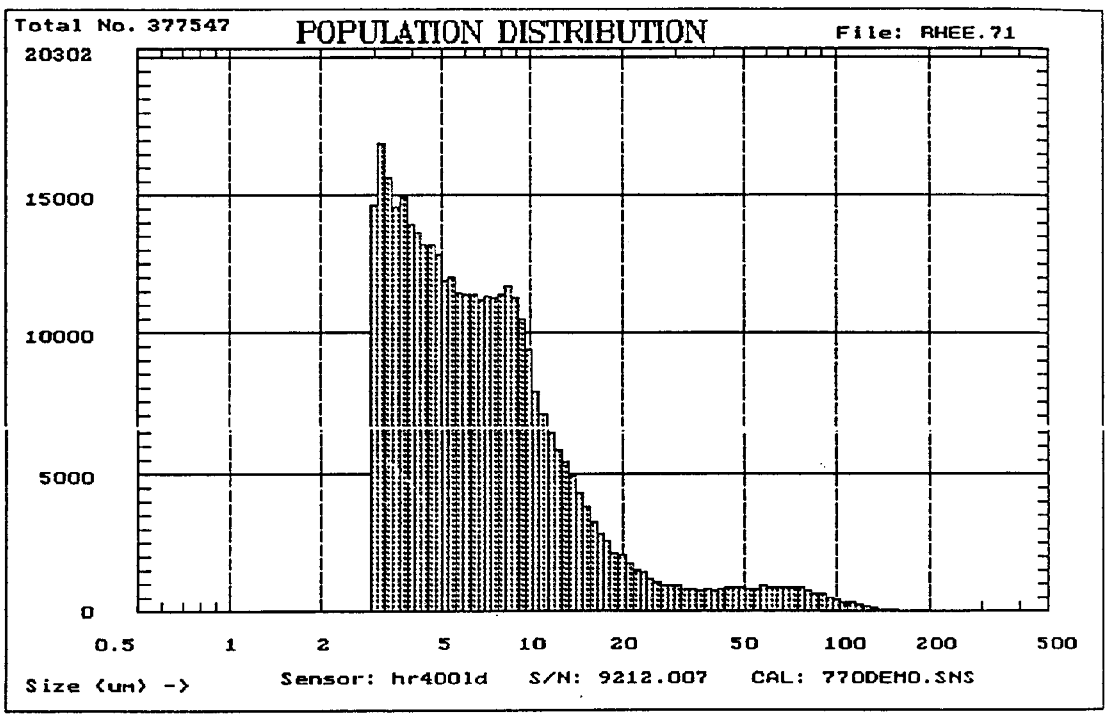


File Name: RHEE.71

Figure 14.